(19)

**Europäisches Patentamt**
**European Patent Office**
**Office européen des brevets**

(11)  **EP 4 742 249 A1**

(12)  # EUROPEAN PATENT APPLICATION
published in accordance with Art. 153(4) EPC

(43) Date of publication:
**13.05.2026 Bulletin 2026/20**

(21) Application number: **24856859.4**

(22) Date of filing: **23.08.2024**

(51) International Patent Classification (IPC):
*G16B 40/20* (2019.01)          *G16B 30/00* (2019.01)
*G16B 50/00* (2019.01)          *G16B 25/00* (2019.01)
*A61K 39/00* (2006.01)          *A61P 35/00* (2006.01)

(52) Cooperative Patent Classification (CPC):
**A61K 39/00; A61P 35/00; G16B 25/00;**
**G16B 30/00; G16B 40/20; G16B 50/00**

(86) International application number:
**PCT/KR2024/012654**

(87) International publication number:
**WO 2025/042248 (27.02.2025 Gazette 2025/09)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **23.08.2023 KR 20230110887**

(71) Applicant: **LG Chem, Ltd.**
**Seoul 07336 (KR)**

(72) Inventors:
• **JEONG, Seihwan**
**Daejeon 34122 (KR)**
• **CHOE, Kyuhong**
**Daejeon 34122 (KR)**
• **KIM, Seunghae**
**Daejeon 34122 (KR)**

• **KIM, Youngmok**
**Daejeon 34122 (KR)**
• **YEU, Yunku**
**Daejeon 34122 (KR)**
• **KIM, Da Eun**
**Daejeon 34122 (KR)**
• **KIM, Hyeongsu**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

Remarks:
The complete document including Reference
Table(s) and the Sequence Listing(s) can be
downloaded from the EPO website

(54)  **METHOD OF SELECTING NEOANTIGEN FOR DEVELOPMENT OF PERSONALIZED CANCER VACCINE**

(57)    Provided are a method of selecting a tumor-specific neoantigen (immunogenic peptide), and a use of the selected tumor-specific neoantigen for preparing a personalized cancer vaccine.

EP 4 742 249 A1

**(Cont. next page)**

【FIG. 3】

**Description**

**[Technical Field]**

CROSS-REFERENCE TO RELATED APPLICATIONS

This application claims the benefit of priority and priority to Korean Patent Application No. 10-2023-0110887, filed on August 23, 2023, with the Korean Intellectual Property Office, the disclosures of which are incorporated herein by reference.

The present disclosure provides a method of selecting neoantigens (tumor-specific immunogenic peptides) and the use of the selected tumor-associated neoantigens for the development of personalized cancer vaccines.

**[Background]**

The term "neoantigen", as used herein, refers to an antigen generated by cancer-specific mutations and, more specifically, to a mutated peptide expressed on the cell surface in a cancer-specific manner that can be recognized as an antigen, including epitopes (surface proteins). Since neoantigens are expressed exclusively in cancer cells, they can be applied to the development of targeted cancer therapies. In the present disclosure, neoantigens are a type of immunogenic peptides and may also be referred to as "tumor-specific neoantigens", "tumor-specific antigens", or "tumor-specific immunogenic peptides".

The term "personalized cancer vaccine" refers to a therapeutic agent or a treatment method in which neoantigens with immune amplification capabilities are selected from cancer-specific antigens expressed in individual patients, produced in the form of proteins or mRNA, and delivered to the patient using lipid-based nanoparticles such as lipid nanoparticles (LNP, Solid Lipid Nanoparticles (SLN), Nanostructured Lipid Carriers (NLC), etc.) or liposome-nucleic acid complexes (e.g., lipoplex, liposomes), activating the patient's immune system to eliminate cancer cells.

In order to mediate the efficacy of personalized cancer vaccines, the key factor is discovering the set of neoantigens specific to each individual patient and selecting and prioritizing immunogenic neoantigens. Immunogenic neoantigens must have a binding affinity with Human Leukocyte Antigen (HLA), which regulates immune responses such as cellular and humoral immunity in response to the antigen stimulation. When the specific neoantigen sequences, that are bound HLA, are recognized by T cell receptors in the body, immune activation against the neoantigens occurs.

However, there are challenges in discovering immunogenic neoantigens applicable to the development of personalized cancer vaccines, as the cancer-specific mutations vary for each patient, and the HLA type expressed differs among individuals. Moreover, the collection and selection of data for training neoantigen AI prediction models that can predict immunogenic neoantigens, as well as the establishment of methods for selecting neoantigens applicable to individual patients in personalized cancer vaccine development, are lacking. This makes it difficult to efficiently utilize neoantigen prediction models. Conventionally, AI prediction models have been developed to measure the binding affinity between immunogenic peptides, which include mutations in cancer cells of patients, and HLA to select neoantigens expected to have anti-cancer efficacy when applied to cancer vaccines. However, it has been difficult to use the derived immunogenic peptides as vaccines, as a high binding affinity between patient epitopes and HLA does not necessarily lead to a strong T cell immune response. Additionally, technologies have been developed to predict T cell immunogenicity by comparing the physicochemical properties of amino acids between peptides containing mutations in cancer cells and nonmutated peptides in non-cancer cells. However, this method, which relies only on amino acid sequence information, fails to reflect the three-dimensional protein structure. As a result, the prediction success rate of immune responses is not high because the HLA-peptide complex, which includes the neoantigen peptides with mutations in cancer cells, must be comprehensively recognized by the T cell receptor to trigger an immune response, and binding affinity alone cannot encompass the diverse immune responses.

**[Disclosure]**

**[Technical Problem]**

One aspect of the present application provides a method for selecting a tumor-specific immunogenic peptide (neoantigen, tumor-specific antigen) (including a prediction method, a selection method, an identification method, or a method for providing information for selection).

The method for selecting a tumor-specific immunogenic peptide may include the steps of: (I) constructing an immunogenic peptide prediction model; and (II) predicting and/or selecting a tumor-specific immunogenic peptide.

The step (I) of constructing an immunogenic peptide prediction model may include the following steps (a) to (d):

(a) obtaining peptide information from a peptide database, wherein the peptide information may include one or more pieces of information selected from sequence information of peptides derived from tumor cells or tumor tissues, amino acid sequence information of peptides derived from normal cells or normal tissues, information on the class I HLA type that binds to the peptides, binding affinity information between the peptides and class I HLA, and/or immunogenicity information of the peptides;

(b) obtaining protein sequence information of the class I HLA from a biological sequence database;

(c) training the information obtained in steps (a) and (b) on a model that can predict the binding affinity score (BA) for HLA to construct a binding affinity prediction model and obtain a binding affinity score; and

(d) training the information obtained in steps (a) and (b) on a model that can predict the immunogenicity score (IMM) to construct an immunogenicity prediction model and obtain an immunogenicity score.

The peptide databases available for use in step (I)-(a) may include one or more peptide databases selected from a group consisting of Immune Epitope Database and Analysis Resource (IEDB), Comprehensive Database of MHC Binding and Non-binding Peptides (MHCBN), but are not limited thereto. Any database containing peptide information, such as amino acid sequence information of peptides derived from humans (normal cells/normal tissues and tumor cells/tumor tissues), and binding information for HLA (e.g., IC50, KD values, etc.) and/or immunogenicity-related information may be used without particular limitation.

The biological sequence databases available for use in step (I)-(b) may include one or more protein databases selected from a group consisting of National Center for Biotechnology Information (NCBI), Protein database, and MHC Motif Atlas, but are not limited thereto. So long as it provides amino acid sequences of HLA proteins, any database may be used for training without particular limitation.

The information obtained from the peptide database and/or biological sequence database may be used for training the prediction models in steps (c) and (d).

The step (II) of predicting and/or selecting tumor-specific immunogenic peptides may include the following steps (1) to (7):

(1) obtaining NGS sequence information from tumor cells or tumor tissues and normal cells or normal tissues derived from a patient;

(2) obtaining sequence information of tumor-specific peptides of at least 7 amino acids in length containing one or more amino acid mutations in the NGS sequence of tumor cells or tumor tissues derived from the patient compared to the NGS sequence of normal cells or normal tissues;

(3) measuring the expression level (TPM; Transcripts per Million) of the tumor-specific peptides or the coding genes of the tumor-specific peptides;

(4) applying the sequence information of the tumor-specific peptides to the binding affinity prediction model constructed in step (c) to measure (confirm, predict) the binding affinity score (BA) to the HLA molecule;

(5) applying the sequence information of the tumor-specific peptides to the immunogenicity prediction model constructed in step (d) to measure (confirm, predict) the immunogenicity score (IMM);

(6) determining the priority ranking of tumor-specific peptides based on the score calculated using the following formula:

$$\text{Neoantigen prioritization score} = \min(\text{TPM},1) * (W1*BA + W2*IMM)/(W1+W2)$$

[wherein 0<W1<1 (or $0.1 \leq W1 \leq 0.9$) and 0<W2<1 (or $0.1 \leq W2 \leq 0.9$) are independently set; "min(TPM,1)" means that the value is used as-is when $0<\text{TPM} \leq 1$, and TPM is set to 1 for calculation when $1 \leq \text{TPM}$ (this is to reduce bias in score interpretation caused by TPM values greater than 1, such as 100 or 1000)]; and

(7) selecting two or more tumor-specific immunogenic peptides based on the determined priority ranking of tumor-specific peptides.

The next-generation sequencing (NGS) sequence information in steps (1) and (2) may be nucleic acid (gene) sequence information and/or sequence information converted into amino acid (protein) sequences. In an embodiment, step (1) may further include a step of converting the obtained NGS sequence into an amino acid sequence after obtaining the NGS sequence information.

The tumor cells or tumor tissues and normal cells or normal tissues in steps (1) and/or (2) may be derived from the same patient, but with no limitations thereto.

The TPM measurement step in step (3) may be performed on tumor cells and/or tumor tissues, and the tumor cells and/or tumor tissues may be obtained from patient-derived samples or available tumor (cancer) cell lines. If the TPM measurement in step (3) is performed on patient-derived tumor cells or tumor tissues, the tumor cells or tumor tissues and normal cells or normal tissues in steps (1) and/or (2) may be derived from the same patient, but with no limitations thereto.

The TPM measurement step in step (3), the binding affinity score measurement step in step (4), and the immunogenicity score measurement step in step (5) are not particularly restricted in their order of execution. For example, they may be performed sequentially regardless of order, or two or more steps may be performed simultaneously, but with no limitations thereto.

The two or more selected tumor-specific immunogenic peptides may be used as final candidate substances for tumor-specific immunogenic peptides (neoantigens).

In an embodiment, the method for selecting tumor-specific immunogenic peptides provided in this description may include the previously described step (II) of predicting and/or selecting tumor-specific immunogenic peptides (i.e., steps (1) to (7)).

In another embodiment, the method for selecting tumor-specific immunogenic peptides may additionally include step (I) of constructing an immunogenic peptide prediction model (i.e., steps (a) to (d)) in addition to step (II) of predicting and/or selecting tumor-specific immunogenic peptides. In this case, the additional step (I) of constructing an immunogenic peptide prediction model may be included prior to step (II) of predicting and/or selecting tumor-specific immunogenic peptides.

For the peptide database used for constructing the prediction model in step (I) of the selection method, data obtained through one or more selection processes chosen from the following i) to iv) may be used:

  i) Deleting data that does not correspond to HLA-A, HLA-B, or HLA-C;
  ii) Deleting data with mutations in HLA molecules;
  iii) Deleting data for peptides with lengths shorter than 7 or longer than 15; and
  iv) Removing amino acid sequences containing amino acid residues other than the 20 human-derived amino acids.

One or more steps of step (I) (steps (a) to (d)) or step (II) (steps (1) to (7)) may be performed by a computational processing device, such as a computer, or a data processing system.

The peptide databases available for training the binding affinity prediction model in step (I) may include one or more peptide databases selected from a group consisting of Immune Epitope Database and Analysis Resource (IEDB), Comprehensive Database of MHC Binding and Non-binding Peptides (MHCBN), but are not limited thereto. Any database containing peptide information, such as amino acid sequence information of human-derived peptides and binding information for HLA (e.g., IC50, KD values, etc.) and/or immunogenicity-related information, may be used for training without particular limitation.

In an embodiment, the step of confirming (measuring, determining) the immunogenicity score (IMM) may include learning data from two or more immunogenicity models related to immune responses when a peptide-MHC (HLA) complex binds to a T-cell receptor (TCR).

The immunogenicity model training data may include two or more types of immunogenicity-related information for peptide candidate sequences obtained from peptide databases or related literature, for example, selected from the group consisting of secretion levels of T-cell-derived cytokines including INF-g, TNF-a, IL-2, IL-1b, etc., T-cell proliferation due to immunogenic activation, chemokine levels including CCL4 and CXCL9, and cytotoxicity values of activated T-cells such as Granzyme B. Specifically, the peptide databases may include one or more peptide databases selected from a group consisting of Immune Epitope Database and Analysis Resource (IEDB) and Comprehensive Database of MHC Binding and Non-binding Peptides (MHCBN), but are not limited thereto. So long as it contains peptide information, such as amino acid sequence information of human-derived peptides and immunogenicity measurement values, any database may be used for training without particular limitation.

The immunogenicity measurement values may be measured by methods including ELISPOT, ELISA, flow cytometry, intracellular staining, binding assays, proliferation assays (e.g., T-cell proliferation assays), cytotoxicity assays (e.g., in vitro/ex vivo/in vivo cytotoxicity assays), and the like.

The immunogenicity model training data may include both qualitatively and quantitatively measured values, with qualitative values statistically converted into numerical data, for example, by beta distribution transformation, compared with negative control values.

Step (II) may be performed using NGS sequence information obtained from a sample (tumor cell/tissue and normal cell/tissue) derived from a personalized subject (mammal, e.g., human) and/or information converted into peptide information.

The tumor-specific immunogenic peptide may be a peptide derived from a cancer cell mutation. In an embodiment, the tumor-specific immunogenic peptide may be derived from a sequence (e.g., nucleic acid sequence obtained from NGS or an amino acid sequence converted therefrom) obtained from genomic sequencing analysis (e.g., next-generation sequencing (NGS), etc.) of a patient-derived sample (e.g., cancer cell). More specifically, the tumor-specific immunogenic peptide may be derived from a sequence (nucleic acid sequence or an amino acid sequence converted therefrom) obtained by genomic sequencing analysis (e.g., next-generation sequencing (NGS), etc.) of a patient-derived sample (e.g., cancer cell), where the sequence differs from that of normal cells (e.g., a sequence with a mutation compared to normal cells).

The HLA molecule may be selected from a group consisting of major histocompatibility complex (MHC) molecules derived from humans. The HLA molecule may include different HLA molecule types and/or different HLA alleles. In an embodiment, the HLA molecule may be an HLA type I molecule.

In the step of constructing the immunogenic peptide prediction model (step (I)), the binding affinity score in step (c) may be obtained by combining (ensemble) predicted values trained using two or more methods based on the amino acid sequence information of the immunogenic peptide and the HLA molecule. The amino acid sequence information of the HLA molecule may be obtained from the peptide database described earlier, but with no limitations thereto.

The immunogenicity score in step (d) may be obtained based on the amount of cytokine secreted when the immunogenic peptide is treated with a sample containing immune cells. The sample containing immune cells may be selected from the group consisting of blood, white blood cells, and peripheral blood mononuclear cells (PBMCs), and the cytokine may be selected from one or more of the group consisting of IFN-g, IL-2, TNF-a, and granzyme B, but with no limitations thereto. In an embodiment, the immunogenicity score in step (2) may be determined based on cytokine secretion information when the candidate peptide is treated with immune cells (e.g., PBMCs, etc.) or a sample containing the same. The cytokine secretion information may be obtained from databases selected from a group consisting of IEDB, MHCBN, etc., but with no limitations thereto.

The immunogenicity score may be indirectly measured as the ratio of positive experiments to the total number of experiments. In an embodiment, the IEDB database provides qualitative measurement values, experiment counts (tested), response counts (responded), and frequency as data instead of quantitative measurement values for cytokine secretion, and these can be used as training data for quantifying the immunogenicity score. Actual experimental values can be converted into numerical data by reflecting the ratio of response counts to experiment counts and can be used for retraining the immunogenicity prediction model. This quantification may be performed using one or more methods selected from the group consisting of mean, median, and beta distribution transformation.

The immunogenicity score in step (d) may be obtained by combining (ensemble) predicted values trained using two or more methods based on the peptide amino acid sequence information from the immunogenicity training data, the amino acid sequence corresponding to the HLA molecule of the immune cells, and the cytokine secretion information when the peptide is treated with immune cells.

The combination (ensemble) of steps (c) and (d) may involve selecting two or more prediction models, trained with different methods, that have the smallest prediction error for validation datasets different from the training data, and combining their predicted values. Specifically, the validation dataset may be part of the model training data set aside in advance.

In step (c) (binding prediction modeling and score measurement) and/or step (d) (immunogenicity prediction modeling and score measurement):

- the training may be performed using one or more models selected from commonly used artificial intelligence (AI) learning models, such as Gated Recurrent Unit (GRU), Transformer, Feed Forward Neural-network (FFN), Graph Neural Network (GNN), etc.;
- the training of step (c) (binding affinity score measurement) and step (d) (immunogenicity score measurement) may be performed by combining one learning method selected from those commonly used in the field, either applied or not applied, with the AI model; and
- the binding affinity score and/or immunogenicity score may be the final predicted value obtained by combining (ensemble) the predicted values from each prediction model trained using one or more AI models and one or more learning methods with a conventional ensemble model, and in an embodiment, the final predicted value may be obtained as [(mean of predicted values) - (variance of predicted values)].

In the step (II) of predicting and/or selecting tumor-specific immunogenic peptides, step (1) may be performed through a bioinformatics analysis process comparing the NGS sequences of tumor cells or tumor tissues derived from a patient with the NGS sequences of normal cells or normal tissues derived from the same patient. The tumor cells or tumor tissues and the normal cells or normal tissues may originate from the same patient. The bioinformatics analysis process may include, but is not limited to, the following processes (i) through (vii), and additional steps may be added or removed as needed:

(i) verifying the integrity of NGS data; for example, software such as FastQC may be used, but with no limitations thereto;
(ii) removing low-quality data; for example, software such as Cutadapt may be used, but with no limitations thereto;
(iii) sequence Alignment to the reference genome; for example, the hg19 reference genome and bwa software may be used, but with no limitations thereto;

(iv) verifying and correcting the quality of the sequence alignment results; for example, software such as GATK may be used to apply Indel realignment and Base Quality Score Recalibration (BQSR), but with no limitations thereto;

(v) identifying tumor-specific mutations; for example, software such as GATK, Strelka, Mutect2, etc., may be used, but with no limitations thereto;

(vi) classifying tumor-specific mutations; for example, mutations may be classified using software such as SnpEff, and only missense, insertion, and deletion mutations that affect the peptide sequence may be selected, but with no limitations thereto;

(vii) extracting peptide sequences that are 7 or greater amino acids in length including the tumor-specific mutations and can be generated.

The TPM (transcripts per million) in step (3) is a measure of gene expression during transcription, the process where a gene sequence composed of nucleotides is transcribed to form transcripts. Methods for measuring expression values include measuring mRNA concentration and measuring transcript concentration, but are not limited thereto, and expression values can be measured in various ways. In an embodiment of the present disclosure, the expression value is the number of reads obtained from the NGS results mapped to the transcriptome, and it may be calculated in TPM units, but with no limitations thereto, and can be expressed in other units to indicate the expression value. The method may further include a step of filtering RNA sequences with a TPM (transcripts per million) of 1 or greater from among the mutated nucleotide sequences or the entire RNA sequence.

The expression level of the tumor-specific peptide or a coding gene therefor in step (3) (e.g., TPM, Transcripts Per Million; Li et al. (2010); the proportion of each transcript (mapped NGS read count) within the library is normalized, and the sum of the TPM values in one library equals 1,000,000, allowing comparison across different samples) may indicate the likelihood of the tumor-specific peptide being present in the actual patient sample (e.g., the likelihood, frequency, or extent of the cancer cell's gene mutation being transcribed and translated into a protein). The expression level of the tumor-specific peptide or its coding gene in step (3) may be obtained through sequence analysis of sample data from the patient subject to personalized treatment, and more specifically, it may be calculated by processing microarray or RNA-seq data. In an embodiment, the TPM of the tumor-specific peptide or a coding gene therefor can be calculated by analyzing the patient's RNA-seq data, and tumor-specific peptides with a TPM value of 1 or greater may be given priority. For example, since TPM represents the expression level per million units of RNA-seq data normalized by gene length and data output, using $TPM \geq 1$ for prioritization gives preference to candidate peptides that occupy at least one-millionth of the total RNA-seq data, thereby prioritizing those that are more likely to be present in the actual patient sample.

The prioritization step in step (6) is performed using the following formula:

$$\text{Neoantigen prioritization score} = \min(TPM, 1) * (W1*BA + W2*IMM)/(W1+W2).$$

wherein, W1 and W2 represent the weights of the binding affinity score (BA) and the immunogenicity score (IMM), respectively, and can be independently set within the range of $0<W1<1$ (or $0.1 \leq W1 \leq 0.9$) and $0<W2<1$ (or $0.1 \leq W2 \leq 0.9$), respectively. In an embodiment, W1+W2 may equal 1, but with no limitations thereto.

In an embodiment, the formula may be applied to candidate peptides that satisfy the conditions of $TPM > 1$, $0.6 >$ binding affinity score $(BA) \geq 0.1$, and immunogenicity score $(IMM) \geq 0.25$, but with no limitations thereto.

The selection step of tumor-specific immunogenic peptides (neoantigens) in step (7) may further include selecting one or more tumor-specific immunogenic peptides from the top 2 to 100, 2 to 75, 2 to 50, 2 to 40, 2 to 30, 2 to 20, 2 to 10, 5 to 100, 5 to 75, 5 to 50, 5 to 40, 5 to 30, 5 to 20, 5 to 10, 10 to 100, 10 to 75, 10 to 50, 10 to 40, 10 to 30, 10 to 20, 20 to 100, 20 to 75, 20 to 50, 20 to 40, or 20 to 30 peptides, arranged in descending order based on the results of step (6).

In cases where the neoantigen (tumor-specific immunogenic peptide) prediction and personalized cancer vaccine described in the present disclosure are produced as mRNA, specific consideration for hydrophobicity may not be necessary. However, when producing the cancer vaccine in peptide form or conducting immunogenicity tests, if the hydrophobicity of the peptide is high (for example, a high content of hydrophobic amino acids), it may be impossible to produce the peptide, thus making it necessary to measure the hydrophobicity of the peptide. In an embodiment, such hydrophobicity can be measured by calculating the Gravy (Grand average of hydropathy) score, but with no limitations thereto.

The tumor-specific immunogenic peptides selected through the method for selecting tumor-specific immunogenic peptide, provided in the present disclosure, are peptides (e.g., mutant peptides) that are specifically present in cancer (tumor) samples and absent in normal samples without cancer (tumor). Therefore, the tumor-specific immunogenic peptides can be effectively used in pharmaceutical compositions for the prevention and/or treatment of cancer, such as in the preparation of cancer vaccines, particularly in the production of personalized cancer vaccines for patients based on the selection method.

Another aspect provides an anti-cancer vaccine composition that includes tumor-specific immunogenic peptides (neoantigens) selected by the tumor-associated immunogenic peptide selection method, and/or coding genes (mRNA, DNA, etc.) therefor. The anti-cancer vaccine composition may further include a pharmaceutically acceptable carrier and/or excipient and/or one or more adjuvants, stabilizers, and the like.

Another aspect provides a method for preparing a cancer vaccine, the method including a step of mixing the tumor-specific immunogenic peptide selected by the tumor-specific immunogenic peptide selection method and/or the coding gene therefor (mRNA, DNA, etc.) with a pharmaceutically acceptable carrier and/or one or more adjuvants, stabilizers, and the like.

The carrier and/or excipient and/or one or more adjuvants, stabilizers, and the like may be selected from any pharmaceutically acceptable substances conventionally used in vaccine formulation.

The vaccine may be in the form of a therapeutic or prophylactic vaccine.

Another aspect provides a method for preventing and/or treating cancer, the method a step of administering the tumor-specific immunogenic peptide selected by the tumor-specific immunogenic peptide selection method or the vaccine to a patient in need of cancer prevention and/or treatment.

The treatment of cancer may be combined with other anti-cancer agents and/or surgical resection and/or radiation therapy and/or traditional chemotherapy. The other anti-cancer agents may be selected from any known anti-cancer agents (such as chemical drugs, antibodies, etc.), for example, immune checkpoint inhibitors (such as PD-1 inhibitors and/or PD-L1 inhibitors), but with no limitations thereto.

**[Technical Solution]**

To discover neoantigens with strong immunogenicity among cancer-specific sequence mutations, an AI-driven neoantigen prediction model was developed by combining a Binding Affinity model, which predicts the binding affinity between HLA and the neoantigen sequence, with an Immunogenicity model that predicts the immunogenicity of the neoantigen sequence. The neoantigen prediction model was trained using the Immune Epitope Database and Analysis Resource (IEDB) and improved through *in silico* updates with data from the database.

Through the training of the AI neoantigen prediction model, it became possible to predict candidate neoantigens with immunogenic potential. By applying this prediction model to NGS data of cancer patients, candidate neoantigens were identified. A threshold was set for the neoantigen candidates predicted by the Binding Affinity and Immunogenicity models during the application of the prediction model. Peptides corresponding to the predicted neoantigens were then synthesized for binding affinity assays and immunogenicity assays (e.g., IFN$\gamma$ ELISPOT) to verify the neoantigen prediction accuracy of model and select neoantigens applicable to personalized cancer vaccines.

The structure of one aspect of the present application can be exemplified as follows:

Neoantigen prediction model (Transformer-based Binding Affinity Model + Immunogenicity model);
Selection of input data for the neoantigen prediction model;
Training method for the neoantigen prediction model;
Processing method for patient data sets, including WES (whole exosome sequencing; mutation selection) and RNA-seq (expression level) data;
Neoantigen prioritization (using TPM, BA Score, and IMM Score to confirm the Neoantigen Ranking Score);
Verification of the applicability of predicted neoantigens to personalized cancer vaccines through binding affinity and immunogenicity assays.

Definition of Terms

As used herein, the term "peptide" refers to a substance containing two or more, three or more, four or more, six or more, eight or more, ten or more, 13 or more, 16 or more, 21 or more, and up to 8, 10, 20, 30, 40, 50, or 100 amino acids covalently linked by peptide bonds. The terms "polypeptide" or "protein" may refer to substances with more amino acid residues covalently linked by peptide bonds than in peptides. However, the terms "peptide", "polypeptide", and "protein", as used herein, may be used interchangeably.

As used herein, the term "modification" or "mutation" in relation to a peptide, polypeptide, or protein refers to a sequence that has been modified from its original (parental) sequence, involving an amino acid insertion, addition, deletion, or substitution. For instance, the modification may be an amino acid substitution. In this specification, any sequence change may potentially create a new epitope.

As used herein, the term "immunogenic peptide" refers to a peptide that can induce an immune response, including an epitope.

The term "neoantigen", as used herein, refers to a peptide or protein that includes one or more amino acid modifications compared to the parental peptide or protein thereof. These amino acid modifications may result from tumor-specific mutations. In this specification, the neoantigen is a type of immunogenic peptide that includes cancer-specific mutations and may not be present or detectable in normal cells. The neoantigen may also be referred to as a tumor-specific neoantigen, tumor-specific antigen, or tumor-specific immunogenic peptide.

The term "immune response" refers to the body's integrated response to a target, such as an antigen, and may involve cellular immunity or both cellular and humoral immune responses. The immune response can be protective, preventive, or therapeutic.

The term "inducing an immune response" may refer to the absence of an immune response prior to induction or may mean that a baseline immune response existed but is enhanced following induction. Thus, "inducing an immune response" also includes "enhancing an immune response". For instance, after immune response induction, the subject may be protected from diseases such as cancer or the disease condition may be improved, alleviated, or mitigated by the induced immune response.

The terms "cellular immune response" and "cellular response" refer to cell-directed immune responses directed by cells characterized by antigen presentation via class I or class II MHC molecules in conjunction with T cells or T lymphocytes acting as "helpers" or "killers". Helper T cells (also called CD4+ T cells) play a central role in regulating immune responses, while killer cells (CD8+ T cells, cytotoxic T lymphocytes, or CTLs) destroy diseased cells, such as cancer cells, preventing the formation of diseased cell populations. In a preferred embodiment, the present disclosure may involve stimulating an antitumor CTL response against tumor cells expressing one or more tumor-expressed antigens, and/or presenting these tumor-expressed antigens via HLA type I or class I MHC.

The term "antigen" encompasses any substance that elicits an immune response, such as a peptide or protein, by interacting with an antibody or T lymphocytes (T cells). For example, an antigen includes at least one epitope, such as a T cell epitope. Herein, antigens may be molecules that induce a specific immune response following processing (including the cells that express the antigen). An antigen or its T cell epitope may be presented by antigen-presenting cells, including diseased cells such as cancer cells, via MHC molecules, leading to an immune response against the antigen (including the cells expressing the antigen).

As used herein, the terms "tumor-specific antigen", "tumor-specifically expressed antigen", "cancer-specific antigen", and "cancer-specifically expressed antigen" are equivalent in meaning and can be used interchangeably with neoantigen.

The term "immunogenicity" refers to the capacity to induce an immune response, such as in treatment against cancer (e.g., antibody formation).

The term "major histocompatibility complex" (MHC) refers to MHC class I and class II molecules and is associated with the gene complex that occurs in all vertebrates. MHC proteins or molecules are critical for signaling between lymphocytes and antigen-presenting cells or diseased cells during immune responses. MHC proteins or molecules bind peptides and present them for recognition by T-cell receptors. Proteins encoded by MHC are expressed on the cell surface and present both self-antigens (peptide fragments from the cell itself) and non-self-antigens (e.g., fragments from invading microorganisms) to T cells.

The MHC region is divided into three subgroups: class I, class II, and class III. MHC class I proteins contain an $\alpha$-chain and $\beta$2-microglobulin (coded by chromosome 15 and not part of MHC) and present antigen fragments to cytotoxic T cells. MHC class II proteins, found primarily on immune cells, including antigen-presenting cells, contain $\alpha$ and $\beta$ chains and present antigen fragments to helper T cells. The class III region codes for other immune components, such as complement proteins and cytokines.

MHC is polygenic (several MHC class I and MHC class II genes exist) and polymorphic (each gene has multiple alleles).

As used herein, the term "haplotype" refers to the HLA alleles found on a chromosome and encoded proteins. A haplotype can also refer to any allele present at any locus within the MHC.

Each class of MHC may be represented by several loci: for instance,

- for class I, Human Leukocyte Antigen (HLA)-A, HLA-B, HLA-C, HLA-E, HLA-F, HLA-G, HLA-H, HLA-J, HLA-K, HLA-L, HLA-P, HLA-V, etc.; and
- for class II, HLA-DR (HLA-DRA, HLA-DRB1-9, etc.), HLA-DQ (HLA-DQA1, HLA-DQB1, etc.), HLA-DP (HLA-DPA1, HLA-DPB1, etc.), HLA-DM (HLA-DMA, HLA-DMB, etc.), HLA-DO (HLA-DOA, HLA-DOB, etc.).

Herein, the terms "HLA or HLA allele" and "MHC or MHC allele" are used interchangeably.

MHCs exhibit strong polymorphism. A large number of haplotypes, each containing distinct alleles, exist at each genetic locus within the human population. Different polymorphic MHC alleles from both class I and class II have varying peptide specificities. Each allele encodes a protein that binds to peptides with specific sequence patterns.

In an embodiment, MHC molecules may be HLA molecules. HLA molecules can be denoted by their serological type according to the "HLA Dictionary 2008" (John Wiley & Sons A/S; Tissue Antigens 73, 95-170, incorporated by reference herein).

The term "MHC-binding peptide" refers to a peptide that can be processed to produce an MHC class I and/or class II binding peptide. For class I MHC/peptide complexes, the binding peptide is generally 7-12, 7-10, 8-12, or 8-10 amino acids in length, but with no limitations thereto. For class II MHC/peptide complexes, the binding peptide is generally 9-30 or 10-25 amino acids in length, such as 13-18 amino acids, but with no limitations thereto.

In an embodiment, when a peptide is presented directly, without processing, especially without cleavage, the length can be suitable for binding to an MHC molecule, particularly a class I MHC molecule, such as a peptide length of 7-30, 7-20, 7-12, 8-11, 9, or 10 amino acids. In another embodiment, when the peptide is part of a larger entity, such as a vaccine sequence or polypeptide, and is presented after processing, especially cleavage, the length of the peptide produced by processing may be suitable for binding to an MHC molecule, particularly a class I MHC molecule, such as 7-30, 7-20, 7-12, 8-11, 9, or 10 amino acids.

The tumor-specific immunogenic peptides selected by the methods provided herein may bind to class I MHC (HLA) molecules.

The term "epitope" refers to an antigenic determinant within a molecule such as an antigen, i.e., a part or fragment of the molecule recognized by the immune system. The epitope of a protein, such as a tumor antigen, may include a continuous or discontinuous portion of the protein, and its length may be 5 to 100, 5 to 50, 8 to 30, or 10 to 25 amino acids. Herein, the epitope may be a T-cell epitope.

Herein, the epitope may be an "MHC-binding peptide" that binds to MHC molecules on the cell surface.

The term "neo-epitope" refers to an epitope found in cancer cells but not present in reference cells, such as normal non-cancerous cells or germ cells. This includes cases where a corresponding epitope exists in normal cells, but mutations in the cancer cells alter the sequence, creating a neo-epitope.

The term "T-cell epitope", as used herein, refers to a peptide that binds to MHC molecules in a configuration recognized by T-cell receptors. T-cell epitopes generally reside on the surface of antigen-presenting cells.

A T-cell epitope may include an amino acid sequence that substantially corresponds to the amino acid sequence of an antigen fragment. For example, the antigen fragment may be a peptide presented by MHC class I and/or class II molecules.

The T-cell epitope according to the present disclosure may be related to a portion or fragment of an antigen that can stimulate a cellular response, such as an immune response to an antigen or cell characterized by the presentation of the antigen, preferably a disease-related cell, particularly a cancer cell. For instance, the T-cell epitope may stimulate a cellular response to cells characterized by antigen presentation by class I MHC, and may stimulate antigen-reactive cytotoxic T-lymphocytes (CTLs).

The term "antigen processing" or "processing" refers to an immunological process that prepares antigens for presentation through cells, i.e., antigen-presenting cells, to specific T cells by breaking down peptides, polypeptides, or proteins (e.g., a polypeptide is broken down into peptides) and then associating the resulting fragments (or parts of them) with MHC molecules.

The terms "T cell" and "T lymphocyte" are used interchangeably herein and include cytotoxic T cells (CTLs, CD8+ T cells), which are cell-lytic T cells, and T helper cells (CD4+ T cells).

T cells are a type of white blood cell in the lymphocyte group and play a central role in cell-mediated immunity. They are distinguished from other lymphocytes, such as B cells and natural killer cells, by the presence of a specific receptor on their surface called the T-cell receptor (TCR). The thymus is the primary organ responsible for T cell maturation. Several different T cell subsets have been identified, each with unique functions.

T helper cells assist other white blood cells in immunological processes, such as the maturation of B cells into plasma cells and the activation of cytotoxic T cells and macrophages. These cells are also known as CD4+ T cells because they express the CD4 protein on their surface. Helper T cells become activated when they are presented with peptide antigens by MHC class II molecules on the surface of antigen-presenting cells (APCs). Once activated, they rapidly divide and secrete small proteins called cytokines, which regulate or assist active immune responses.

Cytotoxic T cells destroy virus-infected cells and tumor cells and are also involved in transplant rejection. These cells are also known as CD8+ T cells because they express the CD8 glycoprotein on their surface. They recognize their targets by binding to antigens associated with MHC class I, which is present on the surface of almost all cells in the body.

Most T cells possess a T-cell receptor (TCR) that exists as a complex of several proteins. The TCR itself is formed from independent TCRα and TCRβ genes and includes two distinct peptide chains called the α and β chains of the TCR. γδ T cells represent a small subset of T cells that express a distinct TCR on their surface. In γδ T cells, the TCR is composed of one γ chain and one δ chain. This group of T cells is much rarer than αβ T cells (making up about 2% of total T cells).

The first signal for T cell activation is provided when the T-cell receptor binds to a short peptide presented by an MHC molecule on another cell. This ensures that only T cells with a TCR specific to that peptide become activated. The partner cell is typically an antigen-presenting cell (APC), such as professional antigen-presenting cells like dendritic cells in the case of naive responses, but B cells and macrophages are also important APCs.

Herein, a molecule is considered capable of binding to a given target if it has significant affinity for that target and binds to it in a standard assay. "Affinity" or "binding affinity" can be measured by the equilibrium dissociation constant (KD). If a molecule does not have significant affinity for the target or does not bind to the target in a significant way in a standard assay, then the molecule does not (substantially) bind to the target.

Specific activation of CD4+ or CD8+ T cells can be confirmed in various ways. Methods for confirming specific T cell activation include detecting T cell proliferation, cytokine (e.g., lymphokine) production, or the generation of cytolytic activity. In the case of CD4+ T cells, specific T cell activation can be confirmed through T cell proliferation. For CD8+ T cells, specific T cell activation can be confirmed through cytolytic activity.

As used herein, the term "score" generally refers to the result of any test or assay, typically expressed numerically.

Herein, determining the binding affinity score of a given peptide to an MHC molecule involves detecting the peptide's potential binding affinity to the MHC molecule.

The binding affinity score of the peptide to the MHC molecule can be determined using peptide binding prediction tools. For example, the Immune Epitope Database and Analysis Resource (IEDB-AR: http://tools.iedb.org) can be used.

According to the present disclosure, amino acid modifications to be detected for their immunogenicity may arise from mutations in the cellular nucleic acid. Such mutations may also be identified using known sequencing techniques.

In an embodiment, cancer-specific somatic mutations or sequence variations can be detected in the exome of a tumor sample, for instance, across the whole exome. Therefore, the present disclosure may include identifying the signs of cancer mutations in the exome, preferably the entire exome, of one or more cancer cells. In an embodiment, the step of identifying a cancer-specific somatic mutation in a tumor specimen of a cancer patient includes identifying a cancer mutation profile across the exome.

In an embodiment, cancer-specific somatic mutations or sequence variations can be detected in the transcriptome of a tumor sample, for instance, across the entire transcriptome. Thus, the present disclosure may include identifying a sign of a cancer mutation in the transcriptome, such as the entire transcriptome, of one or more cancer cells. In an embodiment, the step of identifying a cancer-specific somatic mutation in a tumor sample of a cancer patient includes identifying a cancer mutation profile across the transcriptome.

In an embodiment, the step of identifying a cancer-specific somatic mutation or sequence variation includes single-cell sequencing of one or more cancer cells, for instance, 2, 3, 4, 5, 6, 7, 8, 9, 10, 11, 12, 13, 14, 15, 16, 17, 18, 19, 20 or more cancer cells. Thus, the present disclosure may include identifying a cancer mutation in one or more cancer cells. In an embodiment, the cancer cell is a circulating tumor cell. Cancer cells, such as circulating tumor cells, may be isolated before performing single-cell sequencing.

In an embodiment, the step of identifying a cancer-specific somatic mutation or sequence variation may be carried out using next-generation sequencing (NGS).

In another embodiment, the step of identifying a cancer-specific somatic mutation or sequence variation may include sequencing a genomic DNA and/or RNA of a tumor sample.

To reveal cancer-specific somatic mutations or sequence variations, it is preferable to compare the sequence information obtained from a tumor sample with reference sequence information, such as nucleic acid sequences from normal non-cancerous cells, including germline cells, that can be obtained from the same patient or other individuals. In an embodiment, normal genomic germline DNA may be obtained from peripheral blood mononuclear cells (PBMCs).

The term "genome" refers to pertaining to a total amount of genetic information contained within the chromosomes of an organism or cell.

The term "exome" refers to the portion of a genome that consists of exons, which are the coding sections of expressed genes in an organism. The exome provides the genetic blueprint used for the synthesis of proteins and other functional gene products. Because the exome represents the most functionally relevant part of the genome, it is likely to contribute significantly to an organism's phenotype. The exome of the human genome is estimated to account for 1.5% of the total genome.

The term "transcriptome" refers to the complete set of RNA molecules, including mRNA, rRNA, tRNA, and other non-coding RNAs, produced in a single cell or group of cells. Herein, the transcriptome may refer to the complete set of RNA molecules produced by a single cell, a cell group, such as a group of cancer cells, or all cells of an organism at a given time.

As used herein, the term "nucleic acid" may refer to deoxyribonucleic acid (DNA) or ribonucleic acid (RNA). Nucleic acids include genomic DNA, cDNA, mRNA, recombinant molecules, and chemically synthesized molecules in accordance with the present disclosure. Nucleic acids can exist in single-stranded or double-stranded forms and as linear or covalently modified molecules.

The term "mutation" refers to a change or variation in a nucleic acid sequence (nucleotide substitution, addition, or deletion) compared to a reference, and/or a change in the amino acid sequence of a peptide (protein) encoded by that nucleic acid sequence. A "somatic mutation" occurs in any cell of the body except germ cells (sperm and eggs), and thus, it is not passed on to offspring. Such changes often (but not always) lead to cancer or other diseases. Preferably, mutations are nonsynonymous. The term "nonsynonymous mutation" refers to a mutation that results in an amino acid change, such as an amino acid substitution, preferably due to a nucleotide substitution.

As used herein, the term "mutation" may include point mutations, indels, fusions, chromothripsis, base editing, and/or RNA edits.

The term "cancer mutation signature" refers to a set of mutations present in cancer cells compared to non-cancer reference cells.

The term "reference" can be used to relate or compare the results obtained using the methods of the present disclosure with those from tumor samples. Generally, "reference" can be based on one or more normal samples, for instance, samples from individuals of the same species without cancer.

So long as it is suitable for detecting mutations, any sequencing method may be employed, including, for example, next-generation sequencing (NGS). To speed up the sequencing step, future generations of sequencing technologies, such as so-called thirdgeneration sequencing, may replace NGS. The term "next-generation sequencing" or "NGS" refers to all new high-throughput sequencing technologies, in contrast to "conventional" sequencing methods such as Sanger chemistry, which reads nucleic acid templates randomly across the entire genome by fragmenting the genome into smaller pieces. These NGS technologies (also known as massively parallel sequencing technologies) can deliver nucleic acid sequence information for entire genomes, exomes, transcriptomes (all transcribed sequences of the genome), or methylomes (all methylated sequences of the genome) in very short timeframes, for example, within 1-2 weeks, 1-7 days, or even 24 hours, and they enable single-cell sequencing approaches. Various commercially available or literature-referenced NGS platforms can be used.

The term "RNA" refers to a molecule that contains at least one ribonucleotide residue and preferably consist solely or substantially of ribonucleotide residues. A "ribonucleotide" pertains to a nucleotide that has a hydroxyl group at the 2'-position of the β-D-ribofuranosyl moiety. The term "RNA" encompasses double-stranded RNA, single-stranded RNA, isolated RNA (e.g., partially or fully purified RNA), essentially pure RNA, synthetic RNA, and recombinantly produced RNA, which may differ from naturally occurring RNA through the addition, deletion, substitution, and/or modification of one or more nucleotides. These modifications may include the addition of non-nucleotide substances at one or more termini or internally within the RNA, and the nucleotides in the RNA molecule may include non-standard nucleotides such as chemically synthesized nucleotides or deoxynucleotides. These modified RNAs may be referred to as analogs or mimics of natural RNA.

The term "RNA" may include "mRNA". The term "mRNA" refers to "messenger RNA" that is generated using a DNA template and codes for peptides or polypeptides. It is also known as a "transcript". Typically, mRNA includes a 5'-UTR, a protein-coding region, and a 3'-UTR. mRNA has a limited half-life both in cells and in vitro. mRNA can be generated through in vitro transcription from a DNA template, and such methods of in vitro transcription are known to those skilled in the art.

The terms "reduce" or "suppress" refer to the capacity to cause an overall reduction of 5% or more, 10% or more, 20% or more, 50% or more, or 75% or more. The term "inhibit" or similar expressions also encompass complete or nearly complete inhibition, such as a reduction to zero or effectively zero.

The terms "increase", "enhance", "promote", or "extend" refer to increases, enhancements, promotion, or extension by at least about 10%, least about 20%, least about 30%, least about 40%, least about 50%, least about 80%, least about 100%, least about 200%, or least about 300%. These terms also refer to increases, enhancements, promotion, or extension from zero or undetectable levels to levels greater than zero or detectable/measurable levels.

The term "vaccine" refers to a pharmaceutical preparation (composition) or product that induces an immune response, particularly a cellular immune response, that recognizes and attacks disease cells, such as cancer cells, or pathogens when administered. Vaccines can be used for the prevention or treatment of diseases. The term "personalized cancer vaccines" or "individualized cancer vaccines" refers to cancer vaccines tailored to the specific environment of an individual cancer patient, adjusted as necessary for the unique needs of the patient's condition.

In an embodiment, the vaccine provided by the present disclosure may include one or more peptides selected as immunogenic peptides according to the method of the present disclosure or a nucleic acid (e.g., RNA) encoding the peptide(s).

The cancer vaccine provided by the present disclosure is designed to provide one or more T cell epitopes that are suitable for stimulating, priming, and/or expanding tumor-specific T cells when administered to a patient. The T cells may target cells expressing the antigen from which the T cell epitope is derived. Therefore, the vaccines described herein may induce or enhance cellular responses, such as cytotoxic T cell activity, against cancer by presenting one or more tumor-related neoantigens via class I MHC. Since the vaccine targets cancer-specific mutations, it may be specific to the patient's tumor.

The vaccine provided by the present disclosure may include one or more T cell epitopes, for example, 2 or more, 5 or more, 10 or more, 15 or more, 20 or more, 25 or more, or up to 60, 55, 50, 45, 40, 35, or 30 T cell epitopes, when administered to a patient. These T cell epitopes may also be referred to as "neo-epitopes" in the present disclosure. Presentation of these epitopes by the patient's cells, especially antigen-presenting cells, preferably induces T cells that target the epitope, thereby enabling the targeting of the patient's tumor, including both primary and metastatic tumors, by recognizing and attacking the same epitope presented on the tumor cells' surface.

The term "tumor" or "tumor disease" preferably refers to the abnormal growth of cells (also called neoplastic cells, tumor cells, or neoplasm cells) that form swellings or lesions. "Tumor cells" refer to abnormal cells that grow persistently through unregulated cellular proliferation, even after the stimuli that initiated the new growth have ceased. Tumors may lack functional connections with normal tissues and are partially or fully disorganized, typically forming distinct tissue masses that can be benign, pre-malignant, or malignant.

Cancer is a class of diseases in which groups of cells exhibit uncontrolled growth (division beyond normal limits), invasion (infiltration and destruction of adjacent tissue), and, sometimes, metastasis (spread to other parts of the body via lymph or blood). These three malignant characteristics distinguish cancer from benign tumors, which are self-limited and do not invade or metastasize. Most cancers form tumors, but some, like leukemia, do not. The terms malignancy, malignant neoplasm, and malignant tumor are essentially synonymous with cancer.

For the purposes of the present disclosure, the terms "cancer" and "cancer disease" are used interchangeably with "tumor" and "tumor disease".

The term "cancer" may refer to carcinomas, adenocarcinomas, blastomas, leukemia, mesotheliomas, melanomas, teratomas, lymphomas, neuroblastomas, gliomas, rectal cancer, endometrial cancer, kidney cancer, adrenal cancer, thyroid cancer, blood cancer, skin cancer, brain cancer, cervical cancer, bowel cancer, liver cancer, colon cancer, stomach cancer, intestine cancer, head and neck cancer, gastrointestinal cancer, lymphoma, esophageal cancer, colorectal cancer, pancreatic cancer, ear, nose, and throat (ENT) cancer, breast cancer, prostate cancer, uterine cancer, ovarian cancer, lung cancer, and others. The cancer may be a primary cancer or a metastatic cancer.

The term "treatment" refers to reducing the size or number of tumors in a subject; halting or slowing the progression of the disease; preventing or delaying the development of new diseases in the subject; reducing the frequency or severity of symptoms and/or recurrences in subjects who currently have or previously had the disease; and/or extending the lifespan of the subject.

"Immunotherapy" relates to a treatment method that involves the activation of a specific immune response. In the context of the present disclosure, terms like "protection", "prevention", "preventive", "preventing", and "protective" refer to preventing or treating the onset and/or spread of a disease in a subject or both, particularly by minimizing or delaying the progression of the disease. For example, an individual at risk of developing cancer would be a candidate for cancer preventive therapy.

Preventive administration of immunotherapy, such as preventive administration of the vaccine of the present disclosure, preferably protects the recipient from disease development. Therapeutic administration of immunotherapy, such as therapeutic administration of the vaccine of the present disclosure, may lead to suppression of the progression/growth of the disease. This includes slowing or halting the progression/growth of the disease, for example, leading to the elimination of the disease.

Immunotherapy may be performed using various techniques where the formulation provided by the present disclosure functions to eliminate diseased cells from the patient. Such elimination may occur as a result of inducing or enhancing an immune response in the patient that is specific to the antigen or the cells expressing the antigen.

The formulation and composition provided by the present disclosure can be used alone or in combination with other treatments, such as surgery, radiation therapy, chemotherapy, and/or bone marrow transplants (autologous, syngeneic, allogeneic, or unrelated).

The terms "immunization" or "vaccination" describe a process of treating a patient with the intent of inducing an immune response for therapeutic or prophylactic purposes.

The term "in vivo" refers to pertaining to situations occurring within a subject's body.

The terms "subject", "individual", or "patient" are used interchangeably and relate to vertebrates, such as mammals. For example, in the context of the present disclosure, mammals may include humans, non-human primates, domesticated animals (e.g., dogs, cats, sheep, cattle, goats, pigs, horses), laboratory animals (e.g., mice, rats, rabbits, guinea pigs), and captive animals in zoos. As used herein, the term "animal" is intended to encompass humans. The term "subject" refers to a patient, i.e., an animal, preferably a human suffering from a disease described herein, or to a biological sample (e.g., cancer cells, blood) isolated there from.

The vaccine composition provided herein may further include an adjuvant. The term "adjuvant" refers to a compound that prolongs, enhances, or accelerates an immune response. Preferably, the composition of the present disclosure exerts its effects without the addition of an adjuvant. However, the composition may also contain a known adjuvant. Adjuvants can include heterogenous groups of compounds such as oil emulsions (e.g., Freund's adjuvant), mineral compounds (e.g., alum), bacterial products (e.g., Bordetella pertussis toxin), liposomes, and immunostimulatory complexes. Examples of adjuvants include monophosphoryl-lipid-A (MPL, SmithKline Beecham), saponins such as QS21 (SmithKline Beecham), DQS21 (SmithKline Beecham; WO 96/33739), QS7, QS17, QS18, and QS-L1, incomplete Freund's adjuvant, complete Freund's adjuvant, vitamin E, Montanide, alum, CpG oligonucleotides, and various oil-in-water emulsions derived from biologically degradable oils such as squalene and/or tocopherol.

The vaccine composition may further include pharmaceutically acceptable substances that stimulate the patient's immune response. For example, such substances may be cytokines. Examples of cytokines include interleukin-12 (IL-12), GM-CSF, and IL-18, which can enhance the protective activity of the vaccine.

The vaccine composition may also include a compound that enhances the immune response. These compounds may include co-stimulatory molecules provided in nucleic acid or protein form, such as B7-1 and B7-2 (also known as CD80 and CD86).

The vaccine composition provided herein may be administered by conventional routes, such as injection or infusion. Administration may be performed, for example, orally, intravenously, intraperitoneally, intramuscularly, subcutaneously, or transdermally. In an embodiment, administration may be performed intranodally, such as by injection into lymph nodes.

The vaccine composition may be administered in a therapeutically effective amount. A "therapeutically effective amount" refers to an amount sufficient to elicit a desired response or effect, either alone or in combination with additional doses. The therapeutically effective amount may vary depending on various factors including the condition to be treated, the severity of the disease, individual patient factors such as age, physiological condition, size and weight, duration of treatment, concomitant therapies (if any), the specific route of administration, and the like.

The vaccine composition may be sterile and contain an effective amount of the active therapeutic substance to produce the desired response or effect.

The term "pharmaceutically acceptable" refers to pertaining to a non-toxic substance that does not interfere with the action of the active ingredient in a pharmaceutical composition. Such substances may include salts, buffering agents, preservatives, carriers, adjuvants, and other auxiliary immunostimulants, such as CpG oligonucleotides, cytokines, chemokines, saponins, GM-CSF, and/or RNA. Where appropriate, other therapeutic active compounds may also be included.

A "carrier" refers to a natural or synthetic organic or inorganic component combined with the active ingredient to facilitate, enhance, or enable the application of the active ingredient. In this context, the term "pharmaceutically acceptable carrier" refers to one or more suitable solid or liquid fillers, diluents, or encapsulating substances that are appropriate for administration to a patient.

The vaccine composition may contain a suitable buffering agent, such as acetate salt, citrate salt, borate salt, or phosphate salt.

The vaccine composition may contain a suitable preservative, such as benzalkonium chloride, chlorobutanol, parabens, and thimerosal.

The vaccine composition is typically provided in a unit dosage form and may be prepared by methods well-known in the art. The vaccine composition of the present disclosure may take the form of injectables, capsules, tablets, lozenges, solutions, suspensions, syrups, elixirs, or emulsions, for example.

Compositions suitable for parenteral administration generally include a sterile aqueous or non-aqueous preparation of the active compound that is isotonic with the recipient's blood. Suitable carriers and solvents include Ringer's solution and isotonic sodium chloride solution. In addition, sterile fixed oils may be used as a solvent or suspension medium.

**[Effects of the Invention]**

When applying the features of the present disclosure, the processing of HLA-type I and mutation variants can be efficiently performed through the processing of NGS data for patients.

In the present disclosure, a method is presented for selecting neoantigens suitable for personalized cancer vaccines by employing AI technology for database learning. The method incorporates a novel application and validation of a *Binding Affinity* model and an *Immunogenicity* model for selecting neoantigen candidates, followed by a new application of the *Neoantigen Prioritization* process.

**[Brief Description of the Drawings]**

FIG. 1 shows the distribution of Binding Affinity (BA) scores and Immunogenicity (IMM) scores.
FIG. 2a schematically illustrates the process of measuring immunogenicity scores according to Example 4.
FIGS. 2b to 2d show the results of confirming immunogenicity through INFg ELISPOT by HLA-I type in Example 4 (2b: A*02:01, 2c: A*24:02, 2d: A*11:01).
FIG. 3 is a schematic illustration of the test process of Example 5.1.
FIG. 4 is a graph of the splenocyte IFNg ELISPOT results obtained in Example 5.1.
FIG. 5 is a schematic illustration of the test process of Example 5.2.
FIG. 6 shows graphs of the splenocyte IFNg ELISPOT results obtained in Example 5.2.
FIG. 7 is a plot of the anti-cancer effect (in vivo) of the neoantigen confirmed in Example 6.

**[Mode for Invention]**

A better understanding of the present disclosure may be obtained through the following examples which are set forth to illustrate, but are not to be construed to limit the present disclosure.

**EXAMPLE 1. Construction of Neoantigen Prediction Model (Model Training) (Neoantigen Prediction Model I)**

Using the human leukocyte antigen (HLA) type I, specifically HLA I-A, B, and C, and peptide amino acid sequence information from the Immune Epitope Database and Analysis Resource (IEDB) (https://www.iedb.org), an artificial intelligence model was prepared to predict the binding affinity and immunogenicity of candidate neoantigen peptides. Specifically, the binding affinity and immunogenicity prediction models consisted of an ensemble of 150 different models, each trained using different model structures, data split methods, transfer learning inclusion, training optimization methods, and stopping criteria. The model structures were chosen between GRU (Gated Recurrent Unit) and Transformer. A 5-Fold Cross Validation method was applied for data splitting. For transfer learning, the binding affinity prediction model used a classifier trained with qualitative data, while the immunogenicity prediction model utilized the binding affinity prediction model that had the smallest prediction error for the validation data. For the training optimization method, either AdamW or Stochastic Weights Averaging (SWA) can be used. Stopping criteria were selected based on the lowest prediction errors from the validation data during the entire training process, and five points were chosen in order of the lowest errors.

The preparation process of the neoantigen prediction model is described in more detail below:

**1.1. Selection of Input Data for Neoantigen Prediction Model**

The necessary data for training the HLA type I model was selected from the IEDB database and transformed for use in the training of the Neoantigen Prediction Model. These data were applied to both the Binding Affinity Model and the Immunogenicity Model. For instance, the data selection process may involve one or more tasks such as deleting data that do not correspond to HLA-A, HLA-B, or HLA-C, removing data with mutations in HLA molecules, excluding peptides with a length of less than 7 amino acids or more than 15 amino acids, and eliminating sequences that include non-human-derived amino acids outside the 20 standard amino acids. For example, the data transformation process may involve one or more tasks such as normalizing binding affinity measurement values (e.g., IC50) to a range between 0 and 1 and converting immunogenicity score data provided as [number of tests, number of responses] into the mean of a beta distribution (L. Guangyuan et al., "Deeplmmuno: deep learning-empowered prediction and generation of immunogenic peptides for T-cell immunity" Briefings in Bioinformatics, 22.6, 2021).

For the training of the Immunogenicity Prediction Model, test methods related with immunogenicity including the levels of cytokines, such as IFNg, IL-2, TNFa, Granzyme A, etc., were selected from among various experimental results in the IEDB database (data on T-cell-derived cytokines, including IFN-g, TNF-a, IL-2, IL-1b, produced by PBMC treatment of each peptide, T-cell proliferation triggered by immunogenic activation, chemokines such as CCL4 and CXCL9, and the cytotoxicity of activated T-cells, accounted for by Granzyme B, etc.).

**1.2. Training Method for Neoantigen Prediction Model**

Using the data selected in Example 1.1, the following steps were performed:
For the Binding Affinity Prediction Model, selection was made of one from two model architectures (GRU (J. Chung et al., https://arxiv.org/abs/1412.3555, 2014) and Transformer (Vaswani, Ashish, et al., "Attention is all you need", Advances in Neural Information Processing Systems 30, 2017))), one from the three training methods, and one from the 5-fold Cross Validation data splitting methods. The model was saved at five different training checkpoints where the prediction error for the Validation dataset was the lowest, resulting in a total of 150 model training sessions (2 X 3 X 5 X 5 = 150). The final binding affinity prediction (Prediction Value 1) was calculated by averaging the prediction values from the 150 trained models and calculating their variance.

Similarly, for the Immunogenicity Prediction Model, selection was made of one from two model architectures (GRU, Transformer), one from the three training methods (including training based on the model with the lowest validation error from the Binding Affinity Model), and one from the 5-fold Cross Validation data splitting methods. and the same approach of saving the model at the five checkpoints with the lowest prediction error for the Validation dataset were applied, leading to a total of 150 model training sessions (2 X 3 X 5 X 5 = 150). The final immunogenicity prediction (Prediction Value 2) was calculated by averaging the predictions from the 150 models and calculating their variance.

In calculating Prediction Value 1 and Prediction Value 2, the final prediction value was determined using [(the average of the 150 model prediction values) - (the variance of the 150 model prediction values)]. This approach was chosen because a smaller variance among the predictions of the 150 models indicates a more stable and reliable prediction.

**EXAMPLE 2: Neoantigen Prediction and Selection Stage**

Neoantigens were selected using the neoantigen prediction model constructed in Example 1, and the selection process proceeded through the following steps:

1) Collection of cancer patient NGS data from public databases;
2) Determination of HLA type and identification of cancer cell-derived peptides using the NGS data of the cancer patient, followed by the calculation of TPM;
3) Calculation of prediction scores using the neoantigen prediction model from Example 1;
4) Calculation of neoantigen prioritization and selection of experimental peptides using the formula from Example 3;
5) Conducting binding affinity and immunogenicity experiments using PBMCs with the same HLA type identified in step 2).

For the verification of the neoantigen prediction model, publicly available data from four melanoma patients who achieved complete response following pembrolizumab treatment (HLA type: A*02:01; see "John Wiley & Sons A/S, Tissue Antigens 73, 95-170") were utilized as patient sample data (hereinafter "patient sample data" or "patient sample dataset"). Verification was conducted with reference to the study: [Hugo, Willy, et al. "Genomic and transcriptomic features of response to anti-PD-1 therapy in metastatic melanoma". Cell 165.1 (2016): 35-44.].

**2.1. Data Preparation for Neoantigen Selection (Processing of Patient-Derived Whole Exome Sequencing and RNA-seq Data)**

For the patient sample dataset, a combination of codes was configured to perform data pre-processing, variant discovery, and call setrefinement, allowing for the extraction of mutation sequences and HLA-type information. Specifically:

1) DNA mutation information was converted into peptide mutation information, and RNA abundance data and HLA type data were concatenated. In addition, a process was carried out to check whether the peptides derived from these mutations were found in known protein sequences of *Homo sapiens* using the SwissProt database from Uniprot.
2) Mutation data was obtained using tools like Mutect2, included in the GATK package developed by Broad Institute, and Strelka, developed by Illumina, which are used to identify cancer-specific genetic mutations. After processing the DNA mutation information, the missense mutations and in-frame deletions at the protein level were organized, and any peptides identified in both Mutect2 and Strelka were unified into a single entry. (Koboldt, D. C. et al., VarScan 2: Somatic mutation and copy number alteration discovery in cancer by exome sequencing. Genome Research 22, 568-576 (2012); Cai, L., Yuan, W., Zhang, Z., He, L. & Chou, K. C., In-depth comparison of somatic point mutation callers based on different tumor next-generation sequencing depth data. Scientific Reports 6, 9, https://doi.org/10.1038/srep36540 (2016); Kim, S. et al., Strelka2: fast and accurate calling of germline and somatic variants. Nature Methods 15, 591-594, https://doi.org/10.1038/s41592-018-0051-x (2018); McKenna, A. et al., The Genome Analysis Toolkit: a MapReduce framework for analyzing next-generation DNA sequencing data. Genome Res. 20, 1297-1303, https://doi.org/10.1101/gr.107524.110 (2010); Cibulskis, K., Lawrence, M. S., Carter, S. L., Sivachenko, A., Jaffe, D., Sougnez, C., & Getz, G. (2013). Sensitive detection of somatic point mutations in impure and heterogeneous cancer samples. Nature Biotechnology, 31(3), 213-219. doi:10.1038/nbt.2514).
3) The hydrophobicity of the peptides was calculated to prepare data for the subsequent production of neoantigens. While hydrophobicity considerations may not be necessary when developing neoantigens for personalized cancer vaccines using mRNA, they become crucial when producing peptide-based cancer vaccines or conducting immunogenicity tests. High hydrophobicity (e.g., a high content of hydrophobic amino acids) can make peptide production infeasible. In this Example, hydrophobicity was evaluated by calculating the Gravy (Grand Average of Hydropathy) score.

**2.2. Neoantigen Selection Using Neoantigen Prediction Model (Binding Affinity and Immunogenicity) (Verification for Personalized Cancer Vaccine Application)**

Using the data prepared in Example 2.1, thresholds for binding affinity and immunogenicity were set to apply the neoantigen prediction model, and the selected neoantigens were verified as follows:

- The binding affinity of the predicted neoantigens was compared to a standard peptide, NY-ESO-1 (New York esophageal squamous cell carcinoma 1 peptide (157-165, SLLMWITQC; synthesized by AnyGen)). The binding affinity of predicted neoantigens was validated by comparing IC50 values thereof against the known standard.

- An IFNγ ELISPOT immunogenicity assay was conducted to evaluate the immunogenicity of the predicted neoantigens.
- Normal PBMCs from an HLA-A*02:01 donor were rested for 24 hours, and candidate peptides (50 μM) were pulsed with IL-2 (10 IU/ml) (72 hours, 37°C, 5% CO2). PBMCs were then washed and transferred to an ELISPOT assay plate, followed by re-stimulation with the same peptide (50 μM, 24 hours, 37°C, 5% CO2). The ELISPOT assay was performed according to Immunospot's protocol, and the experiment was repeated with PBMCs from three additional donors to verify reproducibility and statistical significance.

The sequence used for HLA-A*02:01's alpha chain portion is as follows:

SHSMRYFFTSVSRPGRGEPRFIAVGYVDDTQFVRFDSDAASQRMEPRAPWI

EQEGPEYWDGETRKVKAHSQTHRVDLGTLRGYYNQSEAGSHTVQRMYGCDVGSD

WRFLRGYHQYAYDGKDYIALKEDLRSWTAADMAAQTTKHKWEAAHVAEQLRAYLE

GTCVEWLRRYLENGKETLQRT (SEQ ID NO: 1)

Given the variability in immunogenicity testing due to the nature of cell-based experiments, Min-Max normalization and weighted values (1 Lot detection: x1, 2 Lot detection: x2, 3 Lot detection: x3) were applied to rank neoantigens and establish a method for selecting neoantigens suitable for personalized cancer vaccine application.

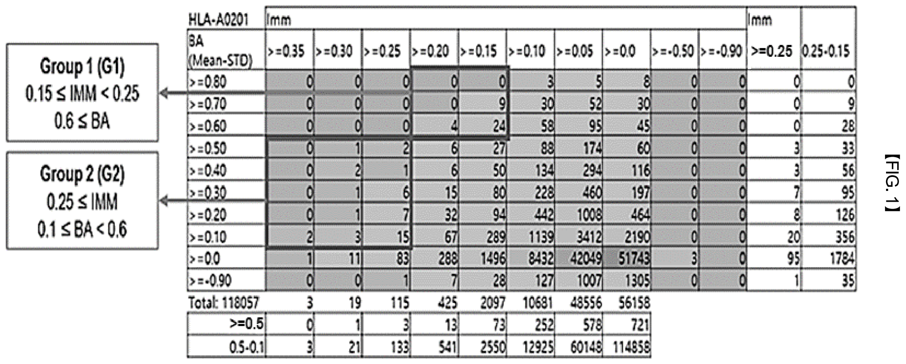

The mutant peptide data of 118,057 peptides derived from patient samples (four melanoma patients treated with pembrolizumab who achieved a complete response, HLA type: A*02:01) were processed using the binding affinity (BA) prediction model and immunogenicity (IMM) prediction model from Example 1. The results are shown in FIG. 1.

Additionally, from the results in FIG. 1, two groups were identified: BA high group (G1; $0.15 \leq$ IMM Score < 0.25 and $0.6 \leq$ BA Score) and IMM high group (G2: IMM Score $\geq 0.25$ and 0.6 > BA Score $\geq 0.1$). The actual binding affinity and immunogenicity for these groups were measured and are shown in Table 1:

TABLE 1

| HLA-I | Binding Affinity (BA) | Immunogenicity (IMM) | Neoantigen Selection Test |
|---|---|---|---|
| G1 | 100% | 5,6% | Four melanoma patients (CR) with pembrolizumab treatment |
| G2 | 49% | 23.6% | |

As shown in Table 1:
When the threshold was set to be $0.15 \leq$ IMM Score < 0.25 and $0.6 \leq$ BA Score (G1), the prediction accuracy for positive binding affinity was 100%.

When the threshold was set to be IMM Score $\geq 0.25$ and 0.6 > BA Score $\geq 0.1$ (G2), the prediction accuracy for positive immunogenicity was 23.6%.

## EXAMPLE 2.3. Measurement of RNA Expression Level (TPM) as Additional Variable

To further evaluate the predicted neoantigens, TPM (RNA expression level; transcripts per million) was measured as an additional variable. TPM indicates the expression rate of RNA sequences from the neoantigen candidates, reflecting the likelihood that the mutations in the patient's cancer cells are translated into actual peptides. However, since the IEDB database does not provide TPM data, it was used for prioritization of the prediction results rather than for model training. To develop evaluation criteria using TPM, publicly available NGS data from cancer patients (TESLA dataset; Wells, Daniel K., et al. "Key parameters of tumor epitope immunogenicity revealed through a consortium approach improve neoantigen prediction". Cell 183.3 (2020): 818-834) were collected to select neoantigen candidates (peptide candidates). The prediction values (Prediction Value 1 and Prediction Value 2) of these neoantigen candidates were calculated using the prediction model (see Example 1.2). The RNA-seq data included in the publicly available NGS data of cancer patients were processed to calculate the RNA expression levels (TPM) of the neoantigen candidates.

**EXAMPLE 3: Evaluation of Selected Neoantigens (Neoantigen Prioritization)**

Regarding the prediction values 1 and 2 obtained from Example 1.2, the optimization of the variables in the Binding Affinity Model and Immunogenicity Model was conducted through Multivariate Linear Regression analysis. Finally, TPM (RNA expression level), Binding Affinity Prediction Score (BA; prediction value 1 from Example 3), and Immunogenicity Prediction Score (IMM; prediction value 2 from Example 3) were confirmed as the variables, and the neoantigen ranking was determined.

The TESLA dataset includes the HLA types and NGS data of 8 patients, 845 peptide sequences as candidate neoantigens derived from those patients, and the immunogenicity experimental results. Among the 845 candidates, 38 peptides were verified to have immunogenicity. To compare the excellence of neoantigen prediction criteria, 20 candidate neoantigens were selected for each of the 8 patients (a total of 160) by applying TPM and varying the weighting of the Binding Affinity Prediction Score (BA) and the Immunogenicity Prediction Score (IMM). The number of neoantigen candidates with verified immunogenicity among the 38 was recorded in Table 2 below (Threshold: TPM (transcripts per million) > 1):

TABLE 2

| Weight Distribution | W1: 1.0 W2: 0.0 | W1: 0.8 W2: 0.2 | W1: 0.5 W2: 0.5 | W1: 0.2 W2: 0.8 | W1: 0.0 W2: 1.0 |
|---|---|---|---|---|---|
| TESLA | 14 | 18 | **26** | 23 | 23 |
| (In Table 2, W1 refers to the weight of BA, and W2 refers to the weight of IMM) | | | | | |

As confirmed in Table 2, when the weights of the Binding Affinity Prediction Score (BA) and the Immunogenicity Prediction Score (IMM) were equal (1:1), the highest number of immunogenic neoantigens was identified.

Based on the above results, the formula for ranking neoantigens was established as follows:

Neoantigen Prioritization Scoring Scheme: [min(TPM,1) * (BA + IMM)/2]

The number of neoantigens detected before and after applying the prioritization formula is shown in Table 3 below:

TABLE 3

| | Patient | Positive | Before Prioritization | After Prioritization |
|---|---|---|---|---|
| Pilot Round | #4 | 1 | 1 | 1 |
| | #8 | 1 | 0 | 1 |
| | #9 | 2 | 1 | 2 |
| | **[310]** | **4** | **2** | **4** |
| Main Round | #1 | 9 | 3 | 7 |
| | #2 | 4 | 3 | 2 |
| | #3 | 13 | 2 | 9 |
| | #12 | 4 | 2 | 2 |
| | #16 | 4 | 1 | 2 |
| | **[535]** | **34** | **11** | **22** |

As shown in Table 3, the application of the prioritization method increased the number of selectable neoantigens, confirming an improvement in neoantigen prediction accuracy.

**EXAMPLE 4. Neoantigen Prediction Model II and Neoantigen Selection**

Referring to the methods described in Examples 1 to 3, patients with melanoma possessing three different HLA types (A*02:01, A*24:02, A*11:01) were selected, and 20 neoantigens were predicted using the neoantigen prediction model. The immunogenicity of the predicted neoantigens was confirmed using IFNγ ELISPOT assays.

More specifically, the criteria for selecting patient data were as follows:

- High Mutation Burden Tumor → Melanoma;
- Complete WES (whole exosome sequencing; Mutation selection), RNA-seq (Expression level) Dataset (converted to peptide sequences for use);
- ICI(Immune Checkpoint Inhibitor) treatment (anti-PD-1 therapy: Pembrolizumab, Nivolumab) → CR(Complete Response), PR(Partial Response), PD(Progressive Disease);
  HLA type: A*02:01, A*24:02, A*11:01 ([HLA-A*02:01]: SHSMRYFFTSVSRPGRGEPRFIAVGYVDDTQFVRFDSD AASQRMEPRAPWIEQEG PEYWDGETRKVKAHSQTHRVDLGTLRGYYNQSEAGSHTVQRMYGCDVGSDWRFL RGYHQYAYDGKDYIALKEDLRSWTAADMAAQTTKHKWEAAHVAEQLRAYLEGTCV EWLRRYLENGKETLQRT (SEQ ID NO: 1), [HLA-A*11:01]: SHSMRYFYTSVSRPGRGEPRFIAVGYVDDTQFVRFDSDAASQRMEPRAPWIE QEG PEYWDQETRNVKAQSQTDRVDLGTLRGYYNQSEDGSHTIQIMYGCDVGPDGRFLR GYRQDAYDGKDYIA LNEDLRSWTAADMAAQITKRKWEAAHAAEQQRAYLEGRCVE WLRRYLENGKETLQRT (SEQ ID NO: 2), [HLA-A*24:02]: SHSMRYFSTSVSRPGRGEPRFIAVGYVDDTQFVRFDSDAASQRMEPRAPWIEQEG PEYWDEETGKV KAHSQTDRENLRIALRYYNQSEAGSHTLQMMFGCDVGSDGRFLR GYHQYAYDGKDYIALKEDLRSWTAADMAA QITKRKWEAAHVAEQQRAYLEGTCVD GLRRYLENGKETLQRT (SEQ ID NO: 3);
- Source: Hugo et al., 2016, Cell 165, 35-44, Riaz et al., 2017, Cell 171, 934-949 (incorporated herein by reference in its entirety).

The neoantigen prediction model applied to the selected patient data was as follows:

Binding Affinity Score (BA) = (Mean Binding Affinity score of the selected patient group) - (Variance of the Binding Affinity Score of the selected patient group);

Immunogenicity Score (IMM) = (Mean Immunogenicity score of the selected patient group) - (Variance of the Immunogenicity Score of the selected patient group)

The BA score was obtained by combining each patient's neoantigen and their unique A*02:01, A*24:02, and A*11:01 using the binding affinity prediction model trained for prediction value 1 in Example 1.2 (see Example 2.1). The IMM score was obtained by predicting the immunogenicity of each patient's neoantigen and their unique HLA types using the immunogenicity prediction model trained for prediction value 2 in Example 1.2 (see Example 2.1).

Using the TPM score (RNA expression level; transcripts per million), the Neoantigen Prioritization Score was calculated (TPM threshold: 1; see Example 3):

$$\text{Prioritization Score} = \min(\text{TPM}, 1) * [(\text{BA} + \text{IMM})/2]$$

Min(TPM,1) means that if TPM > 1, the TPM value is replaced with 1; if TPM < 1, the actual TPM value is used.

The neoantigens were ranked according to the calculated Prioritization Score, and the top 20 peptides were predicted as neoantigens (see Tables 4 to 6).

TABLE 4

| Rank | Sequence | SEQ ID NO | HLA type | Score |
|------|----------|-----------|----------|-------|
| 1 | YLFNFVVNV | 4 | A*02:01 | 0.557830519 |
| 2 | RLAEFIIGM | 5 | A*02:01 | 0.551505094 |
| 3 | YLFNFWNVG | 6 | A*02:01 | 0.501269973 |
| 4 | RLAEFIIGMM | 7 | A*02:01 | 0.499001865 |
| 5 | LLTDIIPDGV | 8 | A*02:01 | 0.494219272 |
| 6 | FLALCADAV | 9 | A*02:01 | 0.493808672 |
| 7 | YIAGILARI | 10 | A*02:01 | 0.490358034 |
| 8 | RLAEFIIGMMG | 11 | A*02:01 | 0.477305298 |
| 9 | YLMVALASV | 12 | A*02:01 | 0.471998357 |

(continued)

| Rank | Sequence | SEQ ID NO | HLA type | Score |
|---|---|---|---|---|
| 10 | RLAEFIIGMMGT | 13 | A*02:01 | 0.459548693 |
| 11 | GLVECLPEI | 14 | A*02:01 | 0.456333267 |
| 12 | AYLFNFWNV | 15 | A*02:01 | 0.448139594 |
| 13 | VLFYLSSAV | 16 | A*02:01 | 0.448117179 |
| 14 | RLFFCFMNV | 17 | A*02:01 | 0.443291682 |
| 15 | CILAVLPSL | 18 | A*02:01 | 0.440896908 |
| 16 | IMFNKGFLA | 19 | A*02:01 | 0.438771916 |
| 17 | KTDPWFLLAV | 20 | A*02:01 | 0.437365302 |
| 18 | SLIGVSFFALPV | 21 | A*02:01 | 0.437040423 |
| 19 | SLWAESLVHV | 22 | A*02:01 | 0.436619991 |
| 20 | FLLAVLGAPV | 23 | A*02:01 | 0.435148758 |

TABLE 5

| Rank | Sequence | SEQ ID NO | HLA type | Score |
|---|---|---|---|---|
| 1 | TFYWTFVNAF | 24 | A*24:02 | 0.455910057 |
| 2 | VFYQFSKLIF | 25 | A*24:02 | 0.403493367 |
| 3 | RMYSSWRLLNL | 26 | A*24:02 | 0.371202482 |
| 4 | TWNVHQQFLW | 27 | A*24:02 | 0.366847255 |
| 5 | RMYSSWRLL | 28 | A*24:02 | 0.36676025 |
| 6 | FYWTFVNAFSIF | 29 | A*24:02 | 0.363375765 |
| 7 | QWILKTRAYFL | 30 | A*24:02 | 0.338967703 |
| 8 | STWNVHQQF | 31 | A*24:02 | 0.338013999 |
| 9 | QWILKTRAYF | 32 | A*24:02 | 0.330919878 |
| 10 | STWNVHQQFLW | 33 | A*24:02 | 0.330000281 |
| 11 | FYWTFVNAF | 34 | A*24:02 | 0.328668696 |
| 12 | STWNVHQQFLW | 35 | A*24:02 | 0.327606417 |
| 13 | FYWTFVNAF | 36 | A*24:02 | 0.324465905 |
| 14 | STWNVHQQFLW | 37 | A*24:02 | 0.320806235 |
| 15 | FYWTFVNAF | 38 | A*24:02 | 0.319582139 |
| 16 | STWNVHQQFLW | 39 | A*24:02 | 0.316823043 |
| 17 | FYWTFVNAF | 40 | A*24:02 | 0.316026847 |
| 18 | STWNVHQQFLW | 41 | A*24:02 | 0.314389744 |
| 19 | FYWTFVNAF | 42 | A*24:02 | 0.313212986 |
| 20 | LYHFSDRMYSSW | 43 | A*24:02 | 0.312662407 |

TABLE 6

| Rank | Sequence | SEQ ID NO | HLA type | Score |
|---|---|---|---|---|
| 1 | STACFHPVTK | 44 | A*11:01 | 0.463350479 |
| 2 | TTFGIQVRYLK | 45 | A*11:01 | 0.436937466 |
| 3 | KIGDFGLATMK | 46 | A*11:01 | 0.42843179 |

(continued)

| Rank | Sequence | SEQ ID NO | HLA type | Score |
|------|----------|-----------|----------|-------|
| 4 | KIGDFGLATEK | 47 | A*11:01 | 0.427919868 |
| 5 | SSSGSINLLK | 48 | A*11:01 | 0.420742704 |
| 6 | AVAVDFQFSK | 49 | A*11:01 | 0.408470424 |
| 7 | KTAPVEWRK | 50 | A*11:01 | 0.392922075 |
| 8 | STSYLQHREK | 51 | A*11:01 | 0.390245612 |
| 9 | VAVDFQFSK | 52 | A*11:01 | 0.382257777 |
| 10 | SAFTIQEYFVK | 53 | A*11:01 | 0.38028147 |
| 11 | SVSNTTVGSK | 54 | A*11:01 | 0.378114721 |
| 12 | RVQTGTIPK | 55 | A*11:01 | 0.377405025 |
| 13 | SSGSINLLK | 56 | A*11:01 | 0.373327626 |
| 14 | GTAAISVTGALK | 57 | A*11:01 | 0.373120201 |
| 15 | STPNFSRLRRY | 58 | A*11:01 | 0.372708181 |
| 16 | RSVLSSGYQK | 59 | A*11:01 | 0.372577947 |
| 17 | VVSPVSLPRR | 60 | A*11:01 | 0.370444394 |
| 18 | AAISVTGALK | 61 | A*11:01 | 0.36963003 |
| 19 | GSQPPSLGRK | 62 | A*11:01 | 0.369425624 |
| 20 | TVYCRYHNY | 63 | A*11:01 | 0.368830969 |

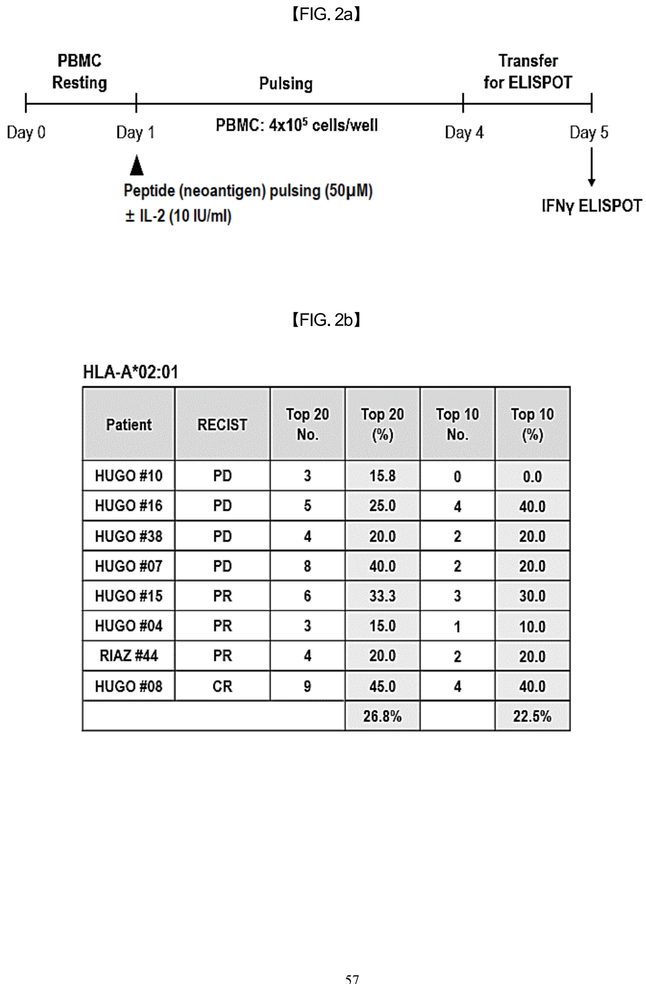

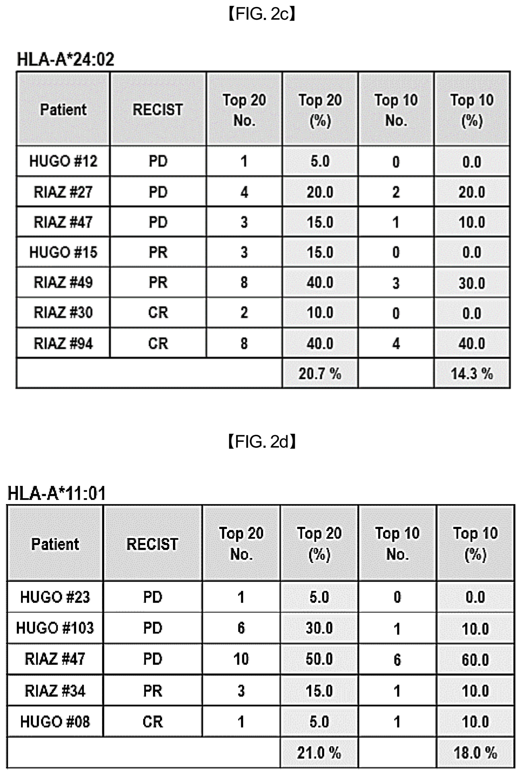

To validate the predicted neoantigens, PBMCs (Peripheral Blood Mononuclear Cells) from three adult donors with known HLA types were obtained and used for immunogenicity assessment via IFNγ ELISPOT (the three donors each had different HLA types, and the obtained PBMCs carried either A*02:01, A*24:02, or A*11:01). The experimental process is schematically illustrated in FIG. 2.

The ELISPOT results obtained were normalized using the following formula for analysis:

$$Normalization = \frac{(X - X(media)}{X(CPI) - X(media)}$$

(Threshold set to >10 to determine immunogenicity positive;

X: Number of spots (ELISPOT) in the test group;

X(media): Number of spots in the media-treated group (untreated group; negative control);

X(CPI): Number of spots in the positive control group treated with protein antigens from CMV, Parainfluenza, and Influenza viruses;

Normalization was performed to maintain consistency across different tests)

For 8 patients (A*02:01), 7 patients (A*24:02), and 5 patients (A*11:01) with matching HLA types, top 20 and top 10 neoantigens were predicted using the neoantigen prediction model described in Examples 1 and 2. Immunogenicity evaluation for the 20 predicted neoantigens was performed using the IFNg ELISPOT method as described in Examples 3 and 4. The number of immunogenic neoantigens in each patient's 20 predicted neoantigens was determined.

The results obtained are shown in Table 7 and FIGS. 2b-2d:

TABLE 7

| HLA-I type | No. of patients | Top20 (%) | Top10 (%) |
|------------|-----------------|-----------|-----------|
| A*02:01 | 8 | 26.8 | 22.5 |
| A*24:02 | 7 | 20.7 | 14.3 |
| A*11:01 | 5 | 21.0 | 18.0 |

Experimental evaluation of immunogenicity for A*02:01 (8 patients), A*24:02 (7 patients), and A*11:01 (5 patients) demonstrated immunogenic neoantigen prediction rates of 26.8%, 20.7%, and 21.0%, respectively.

## EXAMPLE 5. *Ex vivo* Immunogenicity

### 5.1. Human Cell Line (MCF7 cell line) Derived Xenograft Model

The MCF7 breast cancer cell line is a human cancer cell line that expresses HLA-A*02:01. Referring to Example 4, NGS sequencing, including whole exome sequencing (WES; Mutation Selection) and RNA-seq (Expression level), was performed on the MCF7 cell line (ATCC, cat# HTB-22). Using the neoantigen prediction model (BA, IMM, TPM), the top 20 neoantigens were selected, and 20 neoantigens (mRNA) were identified. These were formulated into an mRNA-lipoplex cancer vaccine (see Example A(1) of Patent No. KR 10-2022-0149461 A and KR 10-2022-0149460 A) and administered to A02:01 transgenic mice (C57BL/6 mice engineered to express HLA-A2.1; Taconic biosciences, cat# 8906-F). The vaccine was administered at 60μg per dose, three times weekly (QW), via tail-vein intravenous injection (IV). The immunogenicity was evaluated using splenocyte IFNg ELISPOT.

The immunogenicity assay was conducted as follows: A 96-well ELISPOT plate was coated with anti-IFNg antibodies. After washing the plate with PBS, splenocytes collected from the immunized mice were loaded into each well at $1.0 \times 10^6$ cells per well. For each immune group, cells were plated in triplicate wells. A total volume of 200μl per well was used, with either 10μM of the peptide or media alone for re-stimulation. The plate was then incubated at 37°C for 18 hours. After washing the plate three times with PBST, each well was treated with biotin-labeled mouse IFNg detection antibodies and incubated for 2 hours at room temperature. The cells were washed again, and AP-conjugated streptavidin was added to each well and incubated for 30 minutes at room temperature. The AP reaction was developed using substrate reagent sets (Immunospot). Spot-forming units (SFUs) per well were automatically counted using an ELISPOT reader (CTL ELISPOT reader)..

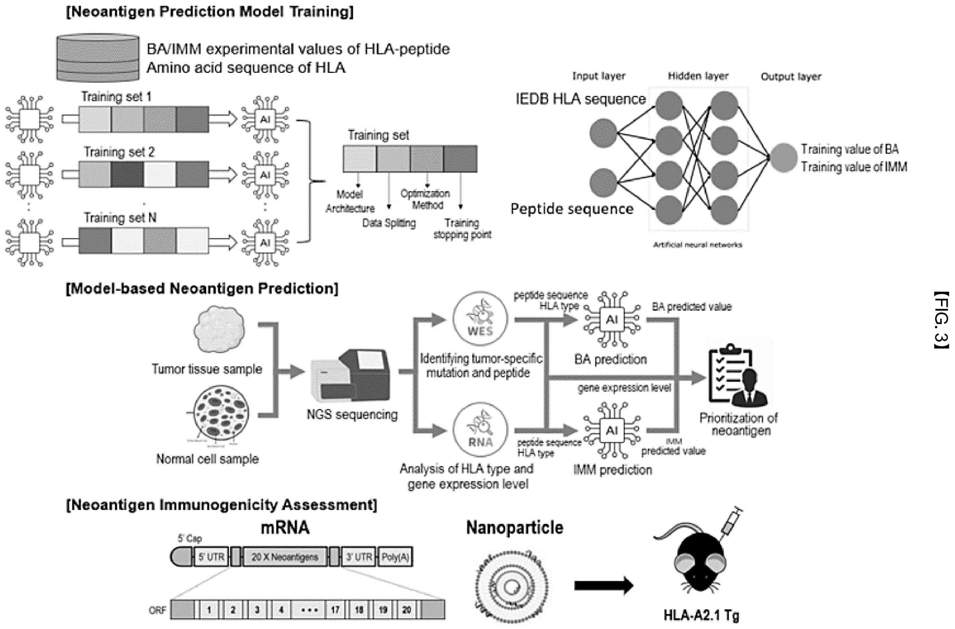

The experimental process, including the vaccine administration, is schematically illustrated in FIG. 3.

The 20 selected neoantigens are shown in Table 8:

TABLE 8

| Rank | Name | Neoantigen | SEQ ID NO |
|------|------|------------|-----------|
| 1 | MCF7-1 | LLFDMTTHSV | 64 |
| 2 | MCF7-2 | FQSFFSLIV | 65 |
| 3 | MCF7-3 | FLGSGVHGGV | 66 |
| 4 | MCF7-4 | YLFFCGHMFHA | 67 |
| 5 | MCF7-5 | ITFEHILAL | 68 |
| 6 | MCF7-6 | NLANEITYI | 69 |
| 7 | MCF7-7 | VLCEPVHGL | 70 |
| 8 | MCF7-8 | MLLESLRHAV | 71 |
| 9 | MCF7-9 | SLFGLGFEEHV | 72 |
| 10 | MCF7-10 | HLLTDELGYV | 73 |
| 11 | MCF7-11 | KLSEASMIV | 74 |
| 12 | MCF7-12 | ALLDMRSLL | 75 |
| 13 | MCF7-13 | GLWEALTPCNV | 76 |
| 14 | MCF7-14 | SLARLHLTV | 77 |
| 15 | MCF7-15 | TLLPLPLLL | 78 |
| 16 | MCF7-16 | HLFWFADLNYRL | 79 |
| 17 | MCF7-17 | STMSFLRSLYL | 80 |
| 18 | MCF7-18 | TLNHEIYEA | 81 |
| 19 | MCF7-19 | ILWGSISFNLAV | 82 |
| 20 | MCF7-20 | MVCGAAFFI | 83 |

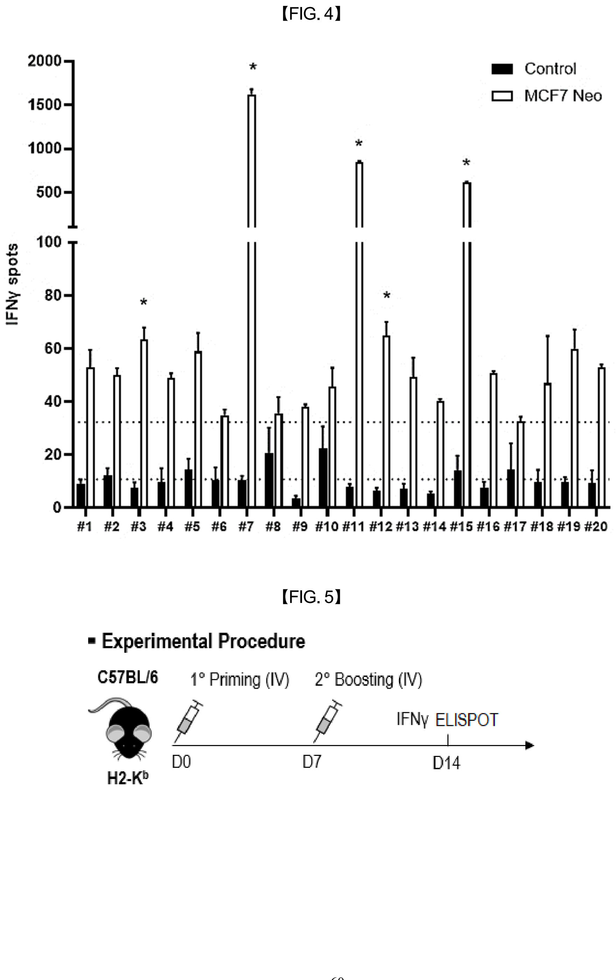

The splenocyte IFNg ELISPOT results obtained are shown in FIG. 4. In FIG. 4, the control refers to the untreated group without neoantigen peptide treatment, and 'MCF7 NEO' refers to the group treated with the mRNA-lipoplex cancer vaccine containing the 20 selected neoantigens. The threshold for considering an anti-cancer effect was set at three times the average ELISPOT count of the untreated group (control). As a result, 25% of the tested 20 neoantigens were predicted to have anti-cancer efficacy, as indicated by the * mark in FIG. 4 (5/20).

### 5.2. B16F10 (Melanoma) AI Model Predicted Neoantigens

Referring to Example 4, NGS was performed on the B16F10 (Mouse Melanoma; ATCC, cat# CRL-6475) cell line (converted to amino acid sequences), and the top 50 and top 20 neoantigens were predicted using the neoantigen prediction model (BA, IMM, TPM). The predicted neoantigens were formulated into mRNA-lipoplex cancer vaccines and administered to C57BL/6 mice (60 μg per dose, two times weekly via IV injection) to evaluate immunogenicity through splenocyte IFNg ELISPOT (see FIG. 5). As a positive control, mTRP2 (Tyrosinase-related protein 2) (Melanoma Tumor-associated Antigen; SVYDFFVWL (SEQ ID NO: 134)) was administered to a mouse model using the same method described previously. The threshold was set to determine the immunogenicity of the predicted neoantigens.

The top 50 neoantigen sequences used in this Example are listed in Table 9:

TABLE 9

| B16F10 Top50 Sequence | | |
|---|---|---|
| **Rank** | **B16F10 Neo Sequence** | **SEQ ID NO** |
| 1 | MSLPFSFL | 84 |
| 2 | ANFIFRQL | 85 |
| 3 | STYKLTNL | 86 |
| 4 | NMYGFRNV | 87 |
| 5 | VIYSHLQV | 88 |
| 6 | SGFRYNVL | 89 |
| 7 | VVMEYENL | 90 |

| | | |
|---|---|---|
| 8 | IVHYFIAL | 91 |
| 9 | VSFAPLVQL | 92 |
| 10 | ATFYFHHPV | 93 |
| 11 | VHYLRLSL | 94 |
| 12 | IGIFFQMRV | 95 |
| 13 | VAHVAAFL | 96 |
| 14 | VFLRVRAL | 97 |
| 15 | TIFMMLRL | 98 |
| 16 | KGFLYSAL | 99 |
| 17 | SSVLFKGL | 100 |
| 18 | MQFYLRHL | 101 |
| 19 | ARMMFSGL | 102 |
| 20 | KSLYYASCL | 103 |
| 21 | LVFVVGRL | 104 |
| 22 | SIGVYIPL | 105 |
| 23 | TVYRCGHL | 106 |
| 24 | SLFTLHQSV | 107 |
| 25 | FTFVYPTI | 108 |

(continued)

| | | |
|---|---|---|
| 26 | CMLRYQTCL | 109 |
| 27 | VCFDYTFAL | 110 |
| 28 | LNVYYLPL | 111 |
| 29 | VPYAVFPPL | 112 |
| 30 | VSFANAW | 113 |
| 31 | VVPSFSAEI | 114 |
| 32 | CNFCYQRNQL | 115 |
| 33 | QMYPAFKRV | 116 |
| 34 | ILVSTYRPL | 117 |
| 35 | SFLAGFRPL | 118 |
| 36 | AIYHHASRAI | 119 |
| 37 | IAMGFPVERL | 120 |
| 38 | VLILHAAAL | 121 |
| 39 | SLVAPFGSV | 122 |
| 40 | MYRYFVTL | 123 |
| 41 | VMMDNFDYL | 124 |
| 42 | VTFQAFIDV | 125 |
| 43 | RAFAAVRL | 126 |
| 44 | KNFEFNKQRSIV | 127 |
| 45 | LMYVYEMLGAGL | 128 |
| 46 | CMLRYQTCLV | 129 |
| 47 | AIALHLHL | 130 |
| 48 | TSYSTAAPL | 131 |
| 49 | SMPHYSSPAI | 132 |
| 50 | TIPFYDNPI | 133 |

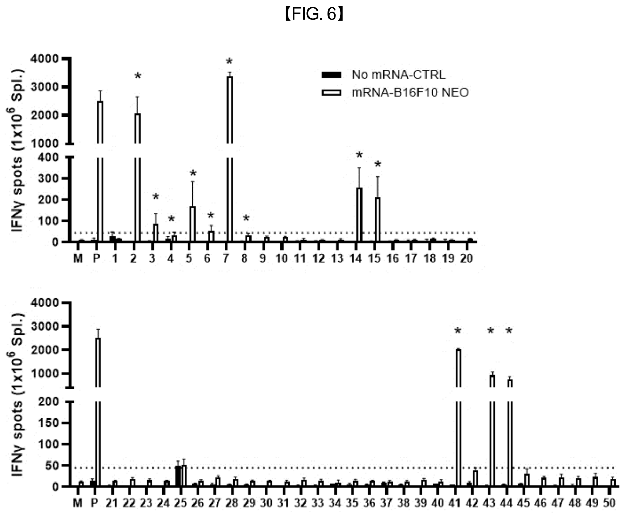

The results obtained are shown in FIG. 6. As depicted in FIG. 6, immunogenicity was confirmed for 45% (9/20) of the top 20 predicted neoantigens (see the upper part of FIG. 6). Of the 12 neoantigens with high immunogenicity among the top 50, 9 were within the top 20, indicating a high likelihood of targeting key neoantigens when focusing on the top 20 (see Table 10).

TABLE 10

| Rank | Positive |
|---|---|
| Top 20 | 9 (45%) |
| Top21~Top 50 | 3 (10%) |
| Total | 12 (24%) |
| (Immunogenicity Threshold: > 3x Media ELISPOT Avg.) | |

**EXAMPLE 6: In vivo Efficacy: Predicted Neoantigen Antitumor Effectiveness**

A total of 5x10$^4$ B16F10 tumor cells were subcutaneously implanted into C57BL/6 mice, and group separation was performed when the tumor size reached 50 mm$^3$. The experimental groups included a group receiving mRNA-lipoplex loaded with the top 10 predicted B16F10 neoantigens (selected from the top 12 neoantigens in Example 5.2), a positive control group receiving mRNA-lipoplex loaded with mTRP2 (Tyrosinase-related protein 2) (Melanoma Tumor-associated Antigen) (SEQ ID NO: 134), and a negative control group receiving lipoplex without any loaded neoantigen mRNA. The mRNA-lipoplex was administered at a dose of 60 μg per injection, three times at five-day intervals, with tumor sizes recorded for three weeks.

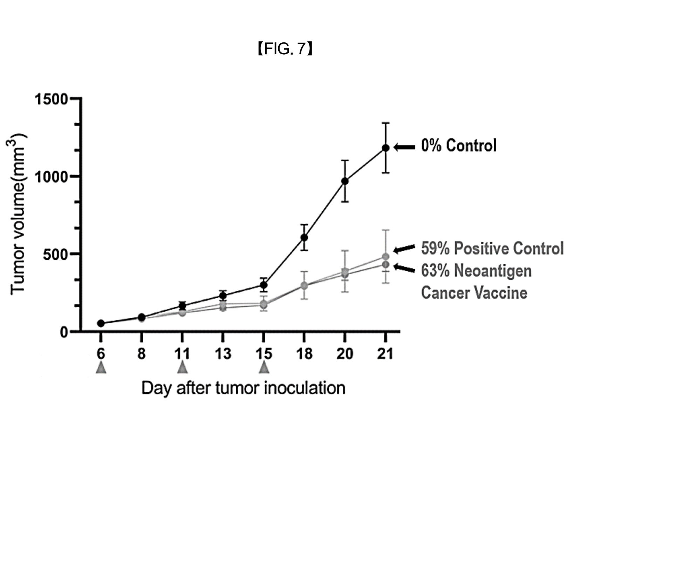

The obtained results (tumor size) are shown in FIG. 7 (Neoantigen Cancer Vaccine: group receiving 10 predicted neoantigens; Positive control: group receiving mTRP2; Control: group receiving lipoplex only (negative control)). As shown in FIG. 7, a 63% reduction in tumor size was observed in the group treated with mRNA-lipoplex containing the top 10 predicted B16F10 neoantigens.

Those of ordinary skill in the art will recognize that the present invention may be embodied in other specific forms without departing from its spirit or essential characteristics. The described embodiments are to be considered in all respects only as illustrative and not restrictive. The scope of the present invention is therefore indicated by the appended claims rather than by the foregoing description. All changes which come within meaning and range of equivalency of the claims are to be embraced within the scope of the present invention.

## Claims

1.  A method for selecting a tumor-specific immunogenic peptide derived from a patient, the method comprising the steps of:

    (1) obtaining NGS sequence information from tumor cells or tumor tissues and normal cells or normal tissues derived from a patient;
    (2) obtaining tumor-specific peptide sequence information of at least 7 amino acids in length, having one or more amino acid variations in the NGS sequence of the tumor cells or tumor tissues compared to the NGS sequence of the normal cells or normal tissues;
    (3) measuring an expression level (TPM; Transcripts per Million) of the tumor-specific peptide or the gene encoding the tumor-specific peptide in the tumor cells or tumor tissues;
    (4) applying the tumor-specific peptide sequence information to a binding affinity prediction model and determining a binding affinity score (BA) to an HLA molecule;
    (5) applying the tumor-specific peptide sequence information to an immunogenicity prediction model and determining an immunogenicity score (IMM);
    (6) determining a priority rank of a tumor-related peptide based on the following formula:

    Neoantigen prioritization score = min(TPM, 1) * (W1*BA + W2*IMM)/(W1+W2) [0<W1<1, 0<W2<1];

    and
    (7) selecting two or more tumor-specific immunogenic peptides based on the determined priority rank of the tumor-specific peptides.

2.  The method of claim 1, further comprising the steps of:

    (a) obtaining peptide sequence information derived from tumor cells or tumor tissues and normal cells or normal tissues from a peptide database;
    (b) obtaining class I HLA peptide sequence information from a biological sequence database;
    (c) constructing a binding affinity prediction model by training the data obtained in steps (a) and (b) on a model capable of predicting a binding affinity score (BA) for HLA, and obtaining a binding affinity score; and
    (d) constructing an immunogenicity prediction model by training the data obtained in steps (a) and (b) on a model capable of predicting an immunogenicity score (IMM), and obtaining an immunogenicity score.

3.  The method of claim 2, wherein the peptide database used for the construction of the prediction model in step (a) undergoes one or more selection processes of the following:

    i) deleting data not corresponding to HLA-A, HLA-B, or HLA-C,
    ii) deleting data with mutations in HLA molecules,
    iii) deleting data with peptide lengths of 7 or less or 15 or more, and
    iv) removing amino acid sequences containing amino acid residues other than the 20 human-derived amino acids.

4. The method of claims 1 to 3, wherein the HLA comprises different HLA types or different HLA alleles.

5. The method of any one of claims 1 to 3, wherein the training in steps (c) and (d) is performed by combining one or more artificial intelligence models and one or more training methods.

6. The method of any one of claims 1 to 3, wherein the binding affinity score is determined by an IC50 value or Kd value related to binding with HLA.

7. The method of any one of claims 1 to 3, wherein the immunogenicity score is determined by one or more pieces of information selected from the group consisting of the cytokine secretion from T-cell, T-cell proliferation due to immunogenic activation, chemokine secretion, and the cytotoxicity of activated T-cell, when the candidate peptide is treated with immune cells or a sample containing the immune cells.

8. The method of claim 7, wherein the sample containing the immune cells is selected from the group consisting of blood, white blood cells, and peripheral blood mononuclear cells (PBMC), and the information is selected from at least two of the group consisting of IFN-g, IL-2, TNF-a, CCL4, CXCL9, and granzyme B.

9. The method of any one of claims 1 to 3, wherein the expression level of the candidate peptide or a coding gene therefor in the patient-derived sample in step (3) is obtained through RNA sequencing from the patient sample data.

10. The method of any one of claims 1 to 3, wherein the tumor-specific immunogenic peptide derived from the patient is used for production of a personalized cancer vaccine for the patient.

11. An anti-cancer vaccine composition comprising:

the tumor-specific immunogenic peptide selected by the method of any one of claims 1 to 3 or a nucleic acid molecule encoding the peptide; and
a pharmaceutically acceptable carrier.

12. A method of preparing an anti-cancer vaccine, the method comprising a step of mixing:

the tumor-specific immunogenic peptide selected by the method of any one of claims 1 to 3 or a nucleic acid molecule encoding the peptide; and
a pharmaceutically acceptable carrier.

【FIG. 1】

| HLA-A0201 BA (Mean-STD) | Imm >=0.35 | >=0.30 | >=0.25 | >=0.20 | >=0.15 | >=0.10 | >=0.05 | >=0.0 | >=-0.50 | >=-0.90 | Imm >=0.25 | 0.25-0.15 |
|---|---|---|---|---|---|---|---|---|---|---|---|---|
| >=0.80 | 0 | 0 | 0 | 0 | 0 | 3 | 5 | 8 | 0 | 0 | 0 | 0 |
| >=0.70 | 0 | 0 | 0 | 0 | 9 | 30 | 52 | 30 | 0 | 0 | 0 | 9 |
| >=0.60 | 0 | 0 | 0 | 4 | 24 | 58 | 95 | 45 | 0 | 0 | 0 | 28 |
| >=0.50 | 0 | 1 | 2 | 6 | 27 | 88 | 174 | 60 | 0 | 0 | 3 | 33 |
| >=0.40 | 0 | 2 | 1 | 6 | 50 | 134 | 294 | 116 | 0 | 0 | 3 | 56 |
| >=0.30 | 0 | 1 | 6 | 15 | 80 | 228 | 460 | 197 | 0 | 0 | 7 | 95 |
| >=0.20 | 0 | 1 | 7 | 32 | 94 | 442 | 1008 | 464 | 0 | 0 | 8 | 126 |
| >=0.10 | 2 | 3 | 15 | 67 | 289 | 1139 | 3412 | 2190 | 0 | 0 | 20 | 356 |
| >=0.0 | 1 | 11 | 83 | 288 | 1496 | 8432 | 42049 | 51743 | 3 | 0 | 95 | 1784 |
| >=-0.90 | 0 | 0 | 1 | 7 | 28 | 127 | 1007 | 1305 | 0 | 0 | 1 | 35 |

Total: 118057

|  | >=0.35 | >=0.30 | >=0.25 | >=0.20 | >=0.15 | >=0.10 | >=0.05 | >=0.0 |
|---|---|---|---|---|---|---|---|---|
|  | 3 | 19 | 115 | 425 | 2097 | 10681 | 48556 | 56158 |
| >=0.5 | 3 | 0 | 3 | 13 | 73 | 252 | 578 | 721 |
| 0.5-0.1 | 3 | 21 | 133 | 541 | 2550 | 12925 | 60148 | 114858 |

Group 1 (G1)
0.15 ≤ IMM < 0.25
0.6 ≤ BA

Group 2 (G2)
0.25 ≤ IMM
0.1 ≤ BA < 0.6

【FIG. 2a】

**PBMC Resting**      **Pulsing**      **Transfer for ELISPOT**

Day 0      Day 1      PBMC: 4x10$^5$ cells/well      Day 4      Day 5

Peptide (neoantigen) pulsing (50μM) ± IL-2 (10 IU/ml)

IFNγ ELISPOT

【FIG. 2b】

## HLA-A*02:01

| Patient | RECIST | Top 20 No. | Top 20 (%) | Top 10 No. | Top 10 (%) |
|---------|--------|------------|------------|------------|------------|
| HUGO #10 | PD | 3 | 15.8 | 0 | 0.0 |
| HUGO #16 | PD | 5 | 25.0 | 4 | 40.0 |
| HUGO #38 | PD | 4 | 20.0 | 2 | 20.0 |
| HUGO #07 | PD | 8 | 40.0 | 2 | 20.0 |
| HUGO #15 | PR | 6 | 33.3 | 3 | 30.0 |
| HUGO #04 | PR | 3 | 15.0 | 1 | 10.0 |
| RIAZ #44 | PR | 4 | 20.0 | 2 | 20.0 |
| HUGO #08 | CR | 9 | 45.0 | 4 | 40.0 |
| | | | 26.8% | | 22.5% |

57

【FIG. 2c】

## HLA-A*24:02

| Patient | RECIST | Top 20 No. | Top 20 (%) | Top 10 No. | Top 10 (%) |
|---------|--------|-----------|-----------|-----------|-----------|
| HUGO #12 | PD | 1 | 5.0 | 0 | 0.0 |
| RIAZ #27 | PD | 4 | 20.0 | 2 | 20.0 |
| RIAZ #47 | PD | 3 | 15.0 | 1 | 10.0 |
| HUGO #15 | PR | 3 | 15.0 | 0 | 0.0 |
| RIAZ #49 | PR | 8 | 40.0 | 3 | 30.0 |
| RIAZ #30 | CR | 2 | 10.0 | 0 | 0.0 |
| RIAZ #94 | CR | 8 | 40.0 | 4 | 40.0 |
| | | | 20.7 % | | 14.3 % |

【FIG. 2d】

## HLA-A*11:01

| Patient | RECIST | Top 20 No. | Top 20 (%) | Top 10 No. | Top 10 (%) |
|---------|--------|-----------|-----------|-----------|-----------|
| HUGO #23 | PD | 1 | 5.0 | 0 | 0.0 |
| HUGO #103 | PD | 6 | 30.0 | 1 | 10.0 |
| RIAZ #47 | PD | 10 | 50.0 | 6 | 60.0 |
| RIAZ #34 | PR | 3 | 15.0 | 1 | 10.0 |
| HUGO #08 | CR | 1 | 5.0 | 1 | 10.0 |
| | | | 21.0 % | | 18.0 % |

【FIG. 3】

【FIG. 4】

【FIG. 5】

## ▪ Experimental Procedure

C57BL/6

H2-K$^b$

1° Priming (IV)    2° Boosting (IV)

IFNγ ELISPOT

D0    D7    D14

【FIG. 6】

【FIG. 7】

## INTERNATIONAL SEARCH REPORT

| International application No. |
| --- |
| **PCT/KR2024/012654** |

| | |
| --- | --- |
| **A.** | **CLASSIFICATION OF SUBJECT MATTER** |

**G16B 40/20**(2019.01)i; **G16B 30/00**(2019.01)i; **G16B 50/00**(2019.01)i; **G16B 25/00**(2019.01)i; **A61K 39/00**(2006.01)i; **A61P 35/00**(2006.01)i

According to International Patent Classification (IPC) or to both national classification and IPC

| | |
| --- | --- |
| **B.** | **FIELDS SEARCHED** |

Minimum documentation searched (classification system followed by classification symbols)

G16B 40/20(2019.01); A61K 35/17(2015.01); A61K 39/00(2006.01); A61P 35/00(2006.01); C12Q 1/6869(2018.01); G01N 33/68(2006.01); G16B 20/30(2019.01); G16B 30/10(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 개인화(personalization), 맞춤 백신(customized vaccine), 종양-특이 펩타이드 (tumor-specific peptide), 신생항원(neoantigen), 시퀀싱(sequencing), 예측 모델(predicted model), 점수(score), 우선순위화 (prioritization)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | KR 10-2021-0064229 A (NEC CORPORATION) 02 June 2021 (2021-06-02)<br>See paragraphs [0012]-[0188]. | 1-3,5-12 |
| A | KR 10-2020-0138418 A (ETUBICS CORPORATION et al.) 09 December 2020 (2020-12-09)<br>See entire document. | 1-3,5-12 |
| A | KR 10-2020-0066305 A (GRITSTONE ONCOLOGY, INC.) 09 June 2020 (2020-06-09)<br>See entire document. | 1-3,5-12 |
| A | KR 10-2020-0014311 A (BIONTECH RNA PHARMACEUTICALS GMBH) 10 February 2020 (2020-02-10)<br>See entire document. | 1-3,5-12 |
| A | US 2022-0378889 A1 (EPIVAX THERAPEUTICS INC.) 01 December 2022 (2022-12-01)<br>See entire document. | 1-3,5-12 |

☐ Further documents are listed in the continuation of Box C.　☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **27 November 2024** | **28 November 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/012654**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/KR2024/012654**

| Box No. II | Observations where certain claims were found unsearchable (Continuation of item 2 of first sheet) |
|---|---|

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☑ Claims Nos.: **4**
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/KR2024/012654**

| Patent document cited in search report | | | Publication date (day/month/year) | Patent family member(s) | | | Publication date (day/month/year) |
|---|---|---|---|---|---|---|---|
| KR | 10-2021-0064229 | A | 02 June 2021 | CN | 112805784 | A | 14 May 2021 |
| | | | | EP | 3844756 | A1 | 07 July 2021 |
| | | | | JP | 2022-504635 | A | 13 January 2022 |
| | | | | JP | 7140280 | B2 | 21 September 2022 |
| | | | | US | 2021-0391031 | A1 | 16 December 2021 |
| | | | | WO | 2020-104539 | A1 | 28 May 2020 |
| KR | 10-2020-0138418 | A | 09 December 2020 | CN | 109890408 | A | 14 June 2019 |
| | | | | EP | 3463440 | A1 | 10 April 2019 |
| | | | | EP | 3735984 | A1 | 11 November 2020 |
| | | | | JP | 2019-517577 | A | 24 June 2019 |
| | | | | JP | 2020-169177 | A | 15 October 2020 |
| | | | | KR | 10-2019-0034504 | A | 02 April 2019 |
| | | | | US | 2020-0282032 | A1 | 10 September 2020 |
| | | | | US | 2021-0138056 | A1 | 13 May 2021 |
| | | | | WO | 2017-205810 | A1 | 30 November 2017 |
| KR | 10-2020-0066305 | A | 09 June 2020 | CN | 111315390 | A | 19 June 2020 |
| | | | | EP | 3679578 | A1 | 15 July 2020 |
| | | | | JP | 2020-532323 | A | 12 November 2020 |
| | | | | JP | 2023-162369 | A | 08 November 2023 |
| | | | | US | 2020-0363414 | A1 | 19 November 2020 |
| | | | | WO | 2019-050994 | A1 | 14 March 2019 |
| KR | 10-2020-0014311 | A | 10 February 2020 | AR | 112601 | A1 | 20 November 2019 |
| | | | | AU | 2018-279117 | A1 | 02 January 2020 |
| | | | | AU | 2018-279117 | A1 | 13 December 2018 |
| | | | | BR | 112019022349 | A2 | 26 May 2020 |
| | | | | CA | 3066308 | A1 | 13 December 2018 |
| | | | | CN | 110741260 | A | 31 January 2020 |
| | | | | CN | 110741260 | B | 08 March 2024 |
| | | | | EP | 3635408 | A1 | 15 April 2020 |
| | | | | IL | 271031 | A | 30 January 2020 |
| | | | | IL | 271031 | D0 | 30 January 2020 |
| | | | | JP | 07-396903 | B2 | 12 December 2023 |
| | | | | JP | 2020-523016 | A | 06 August 2020 |
| | | | | JP | 2024-026224 | A | 28 February 2024 |
| | | | | MA | 49246 | A | 15 April 2020 |
| | | | | RU | 2019139982 | A | 09 July 2021 |
| | | | | RU | 2019139982 | A3 | 09 July 2021 |
| | | | | SG | 10201912475 | A | 27 February 2020 |
| | | | | SG | 11201911618 | A | 30 January 2020 |
| | | | | TW | 201920959 | A | 01 June 2019 |
| | | | | US | 2020-0209251 | A1 | 02 July 2020 |
| | | | | WO | 2018-224166 | A1 | 13 December 2018 |
| | | | | WO | 2018-224405 | A1 | 13 December 2018 |
| US | 2022-0378889 | A1 | 01 December 2022 | EP | 4062177 | A1 | 28 September 2022 |
| | | | | EP | 4062178 | A1 | 28 September 2022 |
| | | | | JP | 2023-522512 | A | 31 May 2023 |
| | | | | JP | 2023-523664 | A | 07 June 2023 |
| | | | | KR | 10-2560750 | B1 | 27 July 2023 |
| | | | | US | 2021-0145951 | A1 | 20 May 2021 |
| | | | | US | 2021-0154280 | A1 | 27 May 2021 |

Form PCT/ISA/210 (patent family annex) (July 2022)

**INTERNATIONAL SEARCH REPORT**
Information on patent family members

International application No.

**PCT/KR2024/012654**

| Patent document cited in search report | Publication date (day/month/year) | Patent family member(s) | | Publication date (day/month/year) |
|---|---|---|---|---|
| | | US 2022-0362365 | A1 | 17 November 2022 |
| | | WO 2021-101962 | A1 | 27 May 2021 |
| | | WO 2021-101965 | A1 | 27 May 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230110887
- WO 9633739 A
- KR 1020220149461 A
- KR 1020220149460 A

### Non-patent literature cited in the description

- **LI et al.** *TPM, Transcripts Per Million*, 2010
- Tissue Antigens. HLA Dictionary 2008. John Wiley & Sons A/S, vol. 73, 95-170
- **L. GUANGYUAN et al.** Deeplmmuno: deep learning-empowered prediction and generation of immunogenic peptides for T-cell immunity. *Briefings in Bioinformatics*, 22 June 2021
- **J. CHUNG et al.** *GRU*, 2014, https://arxiv.org/abs/1412.3555
- **VASWANI, ASHISH et al.** Advances in Neural Information Processing Systems. *Attention is all you need*, 2017, vol. 30
- Tissue Antigens. John Wiley & Sons A/S, vol. 73, 95-170
- **HUGO** ; **WILLY et al.** Genomic and transcriptomic features of response to anti-PD-1 therapy in metastatic melanoma. *Cell*, 2016, vol. 165 (1), 35-44
- **KOBOLDT, D. C et al.** VarScan 2: Somatic mutation and copy number alteration discovery in cancer by exome sequencing. *Genome Research*, 2012, vol. 22, 568-576
- **CAI, L** ; **YUAN, W.** ; **ZHANG, Z.** ; **HE, L.** ; **CHOU, K. C.** In-depth comparison of somatic point mutation callers based on different tumor next-generation sequencing depth data. *Scientific Reports*, 2016, vol. 6, 9, https://doi.org/10.1038/srep36540
- **KIM, S. et al.** Strelka2: fast and accurate calling of germline and somatic variants. *Nature Methods*, 2018, vol. 15, 591-594, https://doi.org/10.1038/s41592-018-0051-x
- **MCKENNA, A. et al.** The Genome Analysis Toolkit: a MapReduce framework for analyzing next-generation DNA sequencing data. *Genome Res.*, 2010, vol. 20, 1297-1303, https://doi.org/10.1101/gr.107524.110
- **CIBULSKIS, K.** ; **LAWRENCE, M. S.** ; **CARTER, S. L.** ; **SIVACHENKO, A.** ; **JAFFE, D.** ; **SOUGNEZ, C.** ; **GETZ, G.** Sensitive detection of somatic point mutations in impure and heterogeneous cancer samples.. *Nature Biotechnology*, 2013, vol. 31 (3), 213-219, doi:10.1038/nbt.2514
- **WELLS** ; **DANIEL K et al.** Key parameters of tumor epitope immunogenicity revealed through a consortium approach improve neoantigen prediction. *Cell*, 2020, vol. 183 (3), 818-834
- **HUGO et al.** *Cell*, 2016, vol. 165, 35-44
- **RIAZ et al.** *Cell*, vol. 171, 934-949